# EUROPEAN PATENT SPECIFICATION

(11) **EP 1 788 093 B1**
(45) Date of publication and mention of the grant of the patent: **15.05.2013**
(21) Application number: 05780216.7
(22) Date of filing: 04.08.2005
(51) Int. Cl.: C12Q 1/68, C12N 15/00, C12M 1/34, C12Q 1/04

(54) **INSTRUMENT FOR DETECTING BACTERIUM, METHOD OF DETECTING BACTERIUM AND KIT FOR DETECTING BACTERIUM**
GERÄT, VERFAHREN UND KIT ZUM NACHWEIS VON BAKTERIEN
INSTRUMENT DE DETECTION DE BACTERIE, PROCEDE DE DETECTION DE BACTERIE ET KIT DE DETECTION DE BACTERIE

(30) Priority: 04.08.2004 JP 2004228669
(43) Date of publication of application: 23.05.2007
(73) Proprietor: Suntory Holdings Limited, Kita-ku, Osaka-shi, Osaka 530-8203 (JP)
(72) Inventor: YAMANAKA, Mikiko, Minami-ku, Kyoto-shi, Kyoto 601-8302 (JP); MORIYA, Shougo, Chiba-shi, Chiba 266-0032 (JP); OSANO, Kaoru, Funabashi-shi, Chiba 273-0031 (JP); OKEGAWA, Takashi, Chiba-shi, Chiba 266-0032 (JP)
(74) Representative: Vossius & Partner
(86) International application number: PCT/JP2005/014716
(87) International publication number: WO 2006/014022

(56) References cited:
- EP-A- 1 273 668
- WO-A1-01/68914
- WO-A1-95/11995
- WO-A1-2005/033337
- JP-A- 2001 095 579
- JP-A- 2002 051 783
- JP-A- 2003 284 559
- JP-A- 2004 097 133
- US-A- 5 840 488
- WILSON KENNETH H ET AL: "High-density microarray of small-subunit ribosomal DNA probes" APPLIED AND ENVIRONMENTAL MICROBIOLOGY, AMERICAN SOCIETY FOR MICROBIOLOGY, US, vol. 68, no. 5, 1 May 2002 (2002-05-01), pages 2535-2541, XP002289520 ISSN: 0099-2240
- LIU W T ET AL: "Optimization of an oligonucleotide microchip for microbial identification studies: a non-equilibrium dissociation approach." ENVIRONMENTAL MICROBIOLOGY OCT 2001, vol. 3, no. 10, October 2001 (2001-10), pages 619-629, XP002508730 ISSN: 1462-2912
- SMALL JACK ET AL: "Direct detection of 16S rRNA in soil extracts by using oligonucleotide microarrays" APPLIED AND ENVIRONMENTAL MICROBIOLOGY, AMERICAN SOCIETY FOR MICROBIOLOGY, US, vol. 67, no. 10, 1 October 2001 (2001-10-01), pages 4708-4716, XP002245295 ISSN: 0099-2240
- ASH CAROL ET AL: "Molecular identification of rRNA group 3 bacilli (Ash, Farrow, Wallbanks and Collins) using a PCR probe test" BIOSIS,, 1 January 1900 (1900-01-01), XP002415454
- DE CLERK E ET AL: 'Isolation, Characterization, and Identification of Bacterial Contaminants in Semifinal Gelatin Extracts.' APPLIED AND ENVIRONMENTAL MICROBIOLOGY. vol. 70, no. 6, June 2004, pages 3664 - 3672, XP002993306

### SEQUENCE LISTING

<110> Suntory Limited Nisshinbo Industries, Inc.
<120> A target bacteria detection instrument, a target bacteria detection method and a target bacteria detection kit
<130> PCT05-0065
<150> JP2004-228669
   <151> 2004-08-04
<160> 57
<170> PatentIn Ver. 2.1
<210> 1
   <211> 20
   <212> DNA
   <213> Bacillus sp.
<400> 1
   agatgggccc gcggcgcatt 20
<210> 2
   <211> 15
   <212> DNA
   <213> Bacillus sp.
<400> 2
   gacccgcggc gcatt 15
<210> 3
   <211> 20
   <212> DNA
   <213> Bacillus sp.
<400> 3
   gggcaacctg cctgtaagac 20
<210> 4
   <211> 21
   <212> DNA
   <213> Brevibacillus sp.
<400> 4
   acatagggaa acttatgcta a 21
<210> 5
   <211> 19
   <212> DNA
   <213> Alicyclobacillus sp.
<400> 5
   gaggagcccg cggcgcatt 19
<210> 6
   <211> 15
   <212> DNA
   <213> Aneurinibacillus sp.
<400> 6
   gaagaaccgc cggga 15
<210> 7
   <211> 20
   <212> DNA
   <213> Geobacillus sp.
<400> 7
   acaccgaaga ccgcatggtc 20
<210> 8
   <211> 18
   <212> DNA
   <213> Paenibacillus sp.
<400> 8
   gacggtacct gagaagaa 18
<210> 9
   <211> 23
   <212> DNA
   <213> Bacillus cereus
<400> 9
   gattaagagc ttgctcttat gaa 23
<210> 10
   <211> 16
   <212> DNA
   <213> Bacillus cereus
<400> 10
   ccgcatggtt cgaaat 16
<210> 11
   <211> 19
   <212> DNA
   <213> Bacillus cereus
<400> 11
   gctagttgaa taagctggc 19
<210> 12
   <211> 20
   <212> DNA
   <213> Bacillus coagulans
<400> 12
   tcgtgcggac cttttaaaag 20
<210> 13
   <211> 17
   <212> DNA
   <213> Bacillus coagulans
<400> 13
   ccgcatggag gaaaaag 17
<210> 14
   <211> 16
   <212> DNA
   <213> Bacillus coagulans
<400> 14
   gccggggaag aacaag 16
<210> 15
   <211> 19
   <212> DNA
   <213> Bacillus sporothermodurans
<400> 15
   ctccgcatgg agagagatt 19
<210> 16
   <211> 20
   <212> DNA
   <213> Bacillus subtilis
<400> 16
   gcatggttca aacataaaag 20
<210> 17
   <211> 14
   <212> DNA
   <213> Bacillus subtilis
<400> 17
   ggctaccact taca 14
<210> 18
   <211> 20
   <212> DNA
   <213> Bacillus subtilis
<400> 18
   gagcttgctc cctgatgtta 20
<210> 19
   <211> 20
   <212> DNA
   <213> Bacillus licheniformis
<400> 19
   tgctccctta ggtcagcggc 20
<210> 20
   <211> 16
   <212> DNA
   <213> Bacillus pumilus
<400> 20
   ggatgaaaga cggttt 16
<210> 21
   <211> 17
   <212> DNA
   <213> Bacillus lentus
<400> 21
   gaatggatgg gagcttg 17
<210> 22
   <211> 16
   <212> DNA
   <213> Bacillus lentus
<400> 22
   agcttgctcc cagaag 16
<210> 23
   <211> 18
   <212> DNA
   <213> Bacillus lentus
<400> 23
   ttctcctgga gaaaggtt 8
<210> 24
   <211> 18
   <212> DNA
   <213> Bacillus lentus
<400> 24
   agcttgctcc cagaagtt 18
<210> 25
   <211> 20
   <212> DNA
   <213> Bacillus lentus
<400> 25
   ctggagaaag gttgaaagac 20
<210> 26
   <211> 20
   <212> DNA
   <213> Bacillus firmus
<400> 26
   gaggaaaagc tgaaagatgg 20
<210> 27
   <211> 20
   <212> DNA
   <213> Bacillus circulans
<400> 27
   gagcggactt taaaagcttg 20
<210> 28
   <211> 17
   <212> DNA
   <213> Alicyclobacillus acidocaldarius
<400> 28
   ttgggccgct gagagag 17
<210> 29
   <211> 20
   <212> DNA
   <213> Bacillus benzoevorans
<400> 29
   gagcggactt aaaaagcttg 20
<210> 30
   <211> 17
   <212> DNA
   <213> Bacillus benzoevorans
<400> 30
   gagcggactt ttgggag 17
<210> 31
   <211> 21
   <212> DNA
   <213> Bacillus megaterium
<400> 31
   taggatcttc tccttcatgg g 21
<210> 32
   <211> 20
   <212> DNA
   <213> Bacillus sphaericus
<400> 32
   tcggctgtcg ctataggatg 20
<210> 33
   <211> 20
   <212> DNA
   <213> Bacillus sphaericus
<400> 33
   aagtacagta gtaactggct 20
<210> 34
   <211> 20
   <212> DNA
   <213> Alicyclobacillus acidoterrestris
<400> 34
   tttcagactg gaataacact 20
<210> 35
   <211> 20
   <212> DNA
   <213> Alicyclobacillus acidoterrestris
<400> 35
   aatacacggg taggcatcta 20
<210> 36
   <211> 16
   <212> DNA
   <213> Alicyclobacillus acidoterrestris
<400> 36
   ggaaagctcc ttgtga 16
<210> 37
   <211> 20
   <212> DNA
   <213> Alicyclobacillus acidocaldarius
<400> 37
   cgcccgcgag gaggcatctt 20
<210> 38
   <211> 19
   <212> DNA
   <213> Paenibacillus validus
<400> 38
   ggggcaacct gtggcttac 19
<210> 39
   <211> 20
   <212> DNA
   <213> Paenibacillus validus
<400> 39
   gctaagaccg gatagctggt 20
<210> 40
   <211> 15
   <212> DNA
   <213> Paenibacillus validus
<400> 40
   cgcctcggag agtaa 15
<210> 41
   <211> 19
   <212> DNA
   <213> Artificial Sequence
<220>
   <223> Description of Artificial Sequence:Artificially Synthesized DNA Sequence
<400> 41
   gagtttgatc ctggctcag 19
<210> 42
   <211> 18
   <212> DNA
   <213> Artificial Sequence
<220>
   <223> Description of Artificial Sequence:Artificially Synthesized DNA Sequence
<400> 42
   gtattaccgc ggctgctg 18
<210> 43
   <211> 20
   <212> DNA
   <213> Bacillus sporothermodurance
<400> 43
   aagagcttgc ttttgatcag 20
<210> 44
   <211> 20
   <212> DNA
   <213> Bacillus sporothermodurance
<400> 44
   cttcgcatga aggagaattg 20
<210> 45
   <211> 20
   <212> DNA
   <213> Bacillus smithii
<400> 45
   agcttgcttt ttgaaagtta 20
<210> 46
   <211> 18
   <212> DNA
   <213> Bacillus smithii
<400> 46
   gataatatct tccttcgc 18
<210> 47
   <211> 15
   <212> DNA
   <213> Alicyclobacillus acidiphilus
<400> 47
   gttcaaggga aggca 15
<210> 48
   <211> 17
   <212> DNA
   <213> Alicyclobacillus acidiphilus
<400> 48
   ccgttgagga aagttgc 17
<210> 49
   <211> 17
   <212> DNA
   <213> Alicyclobacillus acidiphilus
<400> 49
   atgcaacact gatagag 17
<210> 50
   <211> 15
   <212> DNA
   <213> Alicyclobacillus hesperidum
<400> 50
   ggtcacgagg aggca 15
<210> 51
   <211> 17
   <212> DNA
   <213> Alicyclobacillus hesperidum
<400> 51
   gcatcttctt gtgagga 17
<210> 52
   <211> 17
   <212> DNA
   <213> Bacillus fumarioli
<400> 52
   ttgccccttg agattag 17
<210> 53
   <211> 16
   <212> DNA
   <213> Bacillus fumarioli
<400> 53
   tcatcctttc cttcgc 16
<210> 54
   <211> 17
   <212> DNA
   <213> Bacillus acidogenesis
<400> 54
   cttcttcctc cgcatgg 17
<210> 55
   <211> 20
   <212> DNA
   <213> Bacillus pumilus
<400> 55
   ccggatagtt ccttgaaccg 20
<210> 56
   <211> 15
   <212> DNA
   <213> Bacillus sonorensis
<400> 56
   agcgaaccga cggga 15
<210> 57
   <211> 17
   <212> DNA
   <213> Bacillus sonorensis
<400> 57
   tcccttaggt tagcggc 17

## Description

### TECHNICAL FIELD

The present invention relates to a bacteria detection device, a bacteria detection method and a bacteria detection kit. More particularly, the present invention relates to a bacteria detection device in which an oligonucleotide specific to spore-forming, aerobic bacteria is immobilized on a substrate; an extremely accurate and easily operable bacteria detection method; and a bacteria detection kit using this device.

### BACKGROUND ART

Various technologies relating to sanitary control have been researched and developed in the food industry from the viewpoint of safety of the food produced. Food safety in particular has become a subject of strict scrutiny in recent years, and there is a growing interest in improvement of technology.

Microbial control is essential for implementation of sanitary control at food production facilities. In order to accomplish this, it is necessary to test and identify microorganisms. Since testing and identification of microorganisms requires specialized knowledge and technique, they are usually commissioned to various specialized institutions or departments. The commissioned institutions and so on carry out testing and identification of microorganisms by classifying them on the basis of physiological and biochemical properties as well as genetic analyses.

The most important factor in food microbial control is providing accurate test results to food production facilities as quickly as possible. However, the testing, classification and identification methods typically used at present require several days to identify microbial species, and identification of a microorganism, for example, usually takes about several days to one week. Consequently, there is a strong need for the development of a rapid and accurate microbial testing method able to be carried out at food production facilities.

*Bacillus* species (bacteria belonging to the genus *Bacillus*) and other spore-forming, aerobic bacteria in particular are widely distributed throughout nature in the soil, water and air, and many of these species are known to be harmful. For example, among *Bacillus* species, *Bacillus anthracis* and *Bacillus cereus,* which are causative microorganisms of food poisoning, are known to demonstrate pathogenicity in humans.

Known examples of methods for classifying and identifying bacteria include methods based on physiological and biochemical properties, methods based on quinone composition, fungus fatty acid composition and cell wall components, methods based on DNA G+C content, methods based on DNA homology, methods using a DNA probe, and methods involving analysis of nucleotide sequences of 16S ribosomal RNA genes. Recently in particular, it is becoming increasingly common to classify and identify bacteria by using as indicators groups (clusters) obtained by systematic classification based on the nucleotide sequences of 16S ribosomal RNA genes.

Moreover, a known example of a technology for identifying bacteria belonging to spore-forming, aerobic bacteria is disclosed in Japanese Patent No. JP9094700 (publication date: April 8, 1997, Patent Document 1) and Shida, O. et al., Proposal for Two New Genera, Brevibacillus gen. nov. and Aneurinibacillus gen. nov., International Journal of Systematic Bacteriology, 939-946 (1996) (Non-Patent Document 1). In this technology, PCR is applied using DNA having a specific nucleotide sequence present in a 16S ribosomal RNA gene belonging to spore-forming, aerobic bacteria such as *Brevibacillus, Aneurinibacillus* and *Paenibacillus* species as a primer, and using chromosomal DNA obtained from the bacteria to be identified as a template, followed by identifying the bacteria to be identified at the genus level by using amplification of a specific DNA as an indicator.

In addition, a known technology for classifying *Bacillus* species bacteria utilizing 16S ribosomal RNA genes is disclosed in Goto, K. et al., Application of the partial 16S rDNA sequence as an index for rapid identification of species in the genus Bacillus, The Journal of General and Applied Microbiology, 46, 1-8 (2000) (Non-Patent Document 2). Since a roughly 275 bp region on the 5' end of 16S ribosomal RNA genes referred to as the hypervariant (HV) region is a species-specifc region, this technology is able to classify *Bacillus* species bacteria by analyzing the nucleotide sequence of this region.

Wilson et al. (APPLIED AND ENVIRONMENTAL MICROBIOLOGY, AMERICAN SOCIETY FOR MICROBIOLOGY, US,vol. 68, no. 5, 1 May 2002, pages 2535-2541) discloses an microarray with rRNA directed probes to detect Bacillus nucleic acids. JP2003284559 (NASU MASAO; INT REAGENTSCORP) discloses the detection of bacteria, e.g. Bacillus cereus, with an array that comprises 16S rRNA directed probes. Liu et al ( ENVIRONMENTAL MICROBIOLOGY OCT 2001, vol. 3, no. 10, October 2001 ,pages 619-629) discloses an array with 16S rRNA directed probes to detect different Bacillus species.

### DISCLOSURE OF THE INVENTION

However, each of the above-mentioned technologies has difficulty in rapidly and easily identifying microorganisms at food production facilities. Consequently, there is a desire for the development of technology able to be used in pre-shipment testing and so on in the food industry for which there is a particular need for rapid testing.

More specifically, the method disclosed in the above-mentioned Patent Document 1 and Non-Patent Document 1 is inherently not a technology for use in pre-shipment testing and so on in the food industry, but rather places emphasis on bacteria identification itself. Consequently, in this method, although speed is ensured because of use of PCR for detection, since it is a genus-specific method, it is necessary to employ a primer corresponding to each genus, thereby making the procedure complex.

In addition, the method described in Non-Patent Document 2 is still inadequate in terms of speed and simplicity.

Since a rapid and simple microorganism identification method can be utilized not only at food production facilities, but also in the production of pharmaceuticals at and hospitals, research facilities and so on, there is a need for the development of such technology. However, this type of technology has heretofore not been known.

With the foregoing problems in view, an object of the present invention is to provide a technology capable of identifying not only the genus, but also the species of spore-forming, aerobic bacteria, while also enabling rapid and simple identification thereof.

As a result of extensive studies to solve the above-mentioned problems, the inventors of the present invention found a sequence specific to a specific genus or a specific species in nucleotide sequences corresponding to 16S ribosomal RNA genes of typical contaminating bacteria of refreshing beverages and other foods, and also found that target bacteria can be detected and identified both rapidly and accurately by hybridizing with a nucleic acid derived from a test sample using a device in which an oligonucleotide based on this nucleotide sequence is immobilized on a substrate, thereby leading to completion of the present invention.

Accordingly, the present invention includes the inventions described below.
(1) A bacteria detection device for detecting and identifying bacteria belonging to the genus Bacillus and/or Bacillus species in a test sample, wherein an oligonucleotide consisting of a nucleotide sequence of SEQ ID NO:1, an oligonucleotide consisting of the nucleotide sequence of SEQ ID NO:2, and an oligonucleotide consisting of the nucleotide sequence of SEQ ID NO:3 among nucleotide sequences corresponding to a 16S ribosomal RNA gene of bacteria targeted for detection, are immobilized on a substrate; wherein said bacteria detection device is for detecting and identifying said bacteria in a test sample by hybridization between the oligonucleotide and a nucleic acid derived from the test sample.
(2) A bacteria detection device for detecting and identifying bacteria belonging to the genus Bacillus and/or Bacillus species in a test sample, wherein an oligonucleotide consisting of a nucleotide sequence of SEQ ID NO:1, an oligonucleotide consisting of the nucleotide sequence of SEQ ID NO:2, and an oligonucleotide consisting of the nucleotide sequence of SEQ ID NO:3 among nucleotide sequences corresponding to a 16S ribosomal RNA gene of bacteria targeted for detection, are immobilized on a substrate; wherein said bacteria detection device is for detecting and identifying said bacteria in a test sample by hybridization between the oligonucleotide and a nucleic acid derived from the test sample;
   wherein the device further comprises:
   (a) at least one of the oligonucleotides consisting of the nucleotide sequence of SEQ ID NO:9, 10 or 11, for detection of *Bacillus cereus;* and
   (b) at least one of the oligonucleotides consisting of the nucleotide sequence of SEQ ID NO:15, 43 or 44, for detection of *Bacillus sporothermodurans*
(3) The bacteria detection device described in (1) or (2) above, wherein the device has a carbodiimide group or an isocyanate group on the substrate, and a covalent bond is formed as a result of a reaction between the carbodiimide group or the isocyanate group and the oligonucleotide or a linker added to a terminal of the oligonucleotide.
(4) A bacteria detection method for detecting and identifying bacteria belonging to the genus Bacillus and/or Bacillus species in a test sample, comprising the steps of:
   preparing a nucleic acid of the bacteria in the test sample;
   preparing a labeled probe using the nucleic acid as a template;
   hybridizing the labeled probe with an oligonucleotide immobilized on a substrate using a bacteria detection device described in any of (1) to (3) above; and
   detecting a hybridization signal.
(5) The bacteria detection method described in (4) above, wherein the test sample is a food.
(6) The bacteria detection method described in (5) above, wherein the food is a beverage.
(7) The bacteria detection method described in (6) above, wherein the beverage is a refreshing beverage.
(8) A bacteria detection kit for carrying out the bacteria detection method described in any of (4) to (7) above.
(9) A bacteria detection kit for carrying out the bacteria detection method described in any of (4) to (7) above, comprising a reagent used in the hybridization step and the signal detection step.
(10) The bacteria detection kit described in (9) above further comprising a reagent used in the probe preparation step and/or the nucleic acid preparation step.

The bacteria detection device of the present invention allows the detection and identification of bacteria in a test sample by immobilizing on a substrate an oligonucleotide based on a nucleotide sequence specific to a species or genus to which a target bacteria belongs, and hybridizing the oligonucleotide with a nucleic acid derived from the test sample. For this reason, the present invention demonstrates the effect of allowing a target bacteria to be detected and identified both accurately and easily.

In addition, the present invention also demonstrates the effect of being able to carry out comprehensive testing if oligonucleotides based on nucleotide sequences specific to a plurality of bacteria species or genii are immobilized on a substrate.

Moreover, the bacteria detection kit of the present invention demonstrates the effects of improving the ease of use and enabling bacteria to be detected and identified even more easily since it comprises the above-mentioned bacteria detection device and the required reagents.

### BEST MODE FOR CARRYING OUT THE INVENTION

The following provides an explanation of a mode for carrying out the present invention. Furthermore, the present invention is not limited thereto.

### (1) Bacteria Detection Apparatus of the Present Invention

The bacteria detection device of the present invention is an device for detecting and identifying bacteria in a test sample. An oligonucleotide based on a nucleotide sequence specific to a species or genus to which a target bacteria belongs is immobilized on a substrate, and the bacteria in the test sample is detected and identified by hybridizing the oligonucleotide with a nucleic acid derived from the test sample.

### [Substrate]

The material of the substrate used in the bacteria detection device of the present invention may be one capable of stably immobilizing an oligonucleotide. Examples of the material include, but are not limited to, a synthetic resin such as polycarbonate or plastic, and glass. Although there are no particular limitations on the shape of the substrate, a substrate in the form of, for example, a plate or a film can be used preferably.

### [Oligonucleotide Immobilized on the Substrate]

An oligonucleotide immobilized on the substrate of the bacteria detection device of the present invention may be an oligonucleotide based on a nucleotide sequence specific to a species or genus to which a target bacteria belongs. Bacteria belonging to a target species or genus contained in a test sample can be detected by establishing hybridization between the oligonucleotide and a nucleic acid derived from the test sample. Furthermore, the oligonucleotide based on a nucleotide sequence specific to a species or genus to which the target bacteria belongs is hereinafter suitably referred to as a "capture oligonucleotide".

Although a genus- or species-specific nucleotide sequence may be found among nucleotide sequences of the genome of a target bacteria, the above-mentioned specific nucleotide sequence is preferably found among nucleotide sequences corresponding to ribosomal RNA genes of the target bacteria. In particular, since bacterial 16S ribosomal RNA genes are known to have numerous genus- or species-specific nucleotide sequences, a genus- or species-specific nucleotide sequence is particularly preferable found among DNA sequences corresponding to a 16S ribosomal RNA gene. Nucleotide sequences of ribosomal RNA genes can be acquired from a database such as GenBank, EMBL or DDBJ.

The capture oligonucleotide is designed based on the above-mentioned genus- or species-specific nucleotide sequence. Thus, it may be the genus- or species-specific nucleotide sequence itself, or it may contain mutations provided it specifically hybridizes with a nucleic acid prepared from a target bacteria. There are no particular limitations on the locations of mutations.

Although there are no particular limitations on the length (number of nucleotides) of the capture oligonucleotide, it becomes difficult to detect hybridization if the length is excessively short, while non-specific hybridization is permitted if it is excessively long. As a result of conducting extensive studies on optimizing the length of the capture oligonucleotide, the inventors of the present invention determined the standard length to be 12 to 24 nucleotides, and more preferably 13 to 22 nucleotides, although not limited thereto. Since nucleotide length is mainly dependent on the sequence profile (content of a Specific nucleotide, repeats of the same nucleotides), those having satisfactory bonding have been confirmed by the inventors of the present invention to allow specific hybridization even in the case of a short chain.

In the case the capture oligonucleotide has a hairpin structure, loop structure or other steric structure that hinders hybridization with a nucleic acid derived from a test sample, such steric structure can be avoided by substituting one or more of the nucleotides that compose the capture oligonucleotide with inosine or a nucleic acid that does not pair with any of the nucleotides.

There are no particular limitations on the method used to synthesize the capture oligonucleotide, and it may be synthesized using a known method (for example, the method described in Maniatis, T. et al., Molecular Cloning - A Laboratory Manual, Second Edition, Cold Spring Harbor Laboratory Press (1989)). Typically, the capture oligonucleotide is chemically synthesized using a commercially available DNA synthesizer.

In the bacteria detection device of the present invention, in addition to an oligonucleotide based on a nucleotide sequence specific to a genus or species to which a target bacteria belongs, a so-called control capture oligonucleotide is also preferably immobilized on the substrate. A positive control capture oligonucleotide and a negative control capture oligonucleotide are included in the control capture oligonucleotide. A positive control capture oligonucleotide is used to judge whether the amplification reaction proceeds appropriately in the probe preparation step to be described later, and whether hybridization proceeds appropriately. The negative control capture oligonucleotide is used to confirm the absence of non-specific hybridization, namely the absence of false positive hybridization signals. A bacteria detection device in which these positive and negative control capture oligonucleotides are immobilized on the substrate is also included in the present invention.

The positive control capture oligonucleotide may be any oligonucleotide designed based on a nucleotide sequence contained in a probe prepared from a target bacteria. In addition, in the case of simultaneously detecting a plurality of target bacteria using the same bacteria detection device, a positive control capture oligonucleotide may be designed for each target bacteria, or it may be designed based on a nucleotide sequence common to probes prepared from a plurality of target bacteria. In the case there is no nucleotide sequence common to all probes prepared from the target bacteria, the positive control capture oligonucleotide may be designed for several groups each.

Alternatively, an artificial sequence can be designed having the same primer sequence but different from the sequence of a target bacteria, and a portion of this sequence can be used as a positive control capture oligonucleotide. By then preparing a probe using this artificial sequence as a template (this type of probe is herein referred to as a control probe) and adding a probe prepared from a test sample, the amplification reaction in the probe preparation step to be described later and hybridization can be verified to have proceeded favorably.

The negative control capture oligonucleotide is preferably designed to have a nucleotide sequence comprising artificial nucleotide substitutions within a range of one (nucleotide) to less than 20% of the number of nucleotides in the nucleotide sequence of the positive control capture oligonucleotide. The number of nucleotides undergoing nucleotide substitution is determined in the relationship with the hybridization conditions, and the number of nucleotides is selected so as to prevent a probe derived from a target bacteria from hybridizing.

There are no particular limitations on the target bacteria, and the bacteria to be detected is suitably selected from a target test sample. An example of the target bacteria includes those having the possibility of entering a food and contaminating that food. In particular, bacteria having the possibility of entering and contaminating a beverage, and particularly a refreshing beverage, is preferable as target bacteria. The contamination of food by harmful bacteria is an extremely significant problem in terms of public health such as food poisoning. In addition, since this causes turbidity of foods, production of foul odors, deterioration of flavor and other deterioration of product quality, there is a particularly strong desire for the development of a method for rapidly and accurately detecting and identifying these harmful bacteria.

Examples of the above-mentioned target bacteria include bacteria belonging to the genus *Bacillus,* and more specifically, *Bacillus cereus, Bacillus coagulans, Bacillus sporothermodurans, Bacillus subtilis, Bacillus licheniformis, Bacillus pumilus, Bacillus lentus, Bacillus firmus, Bacillus circulans, Bacillus benzoevorans, Bacillus megaterium, Bacillus sphaericus, Bacillus smithii, Bacillus fumarioli, Bacillus acidogenesis* and *Bacillus sonorensis.*

In addition to the above-mentioned bacteria, other examples of target bacteria include bacteria belonging to the genus *Alicyclobacillus,* and more specifically, *Alicyclobacillus acidoterrestris, Alicyclobacillus acidocaldarius, Alicyclobacillus hesperidum, Alicyclobacillus* cycloheptanicus, *Alicyclobacillus herbarius* and *Alicyclobacillus acidiphilus.*

In addition, other examples of target bacteria include bacteria belonging to the genus *Paenibacillus,* and more specifically, *Paenibacillus validus, Paenibacillus illinoisensis, Paenibacillus alvei* and *Paenibacillus polymyxa.*

In addition, still other examples of target bacteria include bacteria belonging to the *genus Aneurinibacillus,* and more *specifically, Aneurinibacillus aneurinolyticus.*

Still other examples of target bacteria include bacteria belonging to the genus *Geobacillus,* and more specifically, *Geobacillus stearothermophilus.*

In addition, other examples of target bacteria include bacteria belonging to the genus *Brevibacillus,* and more specifically, *Brevibacillus brevis, Brevibacillus agri* and *Brevibacillus laterosporus.* However, the target bacteria to be detected are not limited to the above-mentioned examples.

Examples of capture oligonucleotides for detecting and identifying the above-mentioned bacteria include oligonucleotides based on sequences specific to each genus among nucleotide sequences corresponding to a 16S ribosomal RNA gene of bacteria belonging to the genii *Bacillus, Brevibacillus, Alicyclobacillus, Aneurinibacillus, Geobacillus* and *Paenibacillus.*

Specific examples of oligonucleotides based on a sequence specific to the genus *Bacillus* among nucleotide sequences corresponding to a 16S ribosomal RNA gene of bacteria belonging to the genus *Bacillus* include oligonucleotides comprising a nucleotide sequence of SEQ ID NO. 1, 2 or 3 below.
AGATGGGCCCGCGGCGCATT (SEQ ID NO. 1)
GACCCGCGGCGCATT (SEQ ID NO.2)
GGGCAACCTGCCTGTAAGAC (SEQ ID NO. 3)

A specific example of an oligonucleotide based on a sequence specific to the genus *Brevibacillus* among nucleotide sequences corresponding to a 16S ribosomal RNA gene of bacteria belonging to the genus *Brevibacillus* include an oligonucleotide comprising the nucleotide sequence of SEQ ID NO.4 below.
ACATAGGGAAACTTATGCTAA (SEQ ID NO. 4)

A specific example of an oligonucleotide based on a sequence specific to the genus *Alicyclobacillus* among nucleotide sequences corresponding to a 16S ribosomal RNA gene of bacteria belonging to the genus *Alicyclobacillus* includes an oligonucleotide comprising the nucleotide sequence of SEQ ID NO.5 below.
GAGGAGCCCGCGGCGCATT (SEQ ID NO:5)

A specific example of an oligonucleotide based on a sequence specific to the genus *Aneurinibacillus* among nucleotide sequences corresponding to a 16S ribosomal RNA gene of bacteria belonging to the genus *Aneurinibacillus* includes an oligonucleotide comprising the nucleotide sequence of SEQ ID NO. 6 below.
GAAGAACCGCCGGGA (SEQ ID NO. 6)

A specific example of an oligonucleotide based on a sequence specific to the genus *Geobacillus* among nucleotide sequences corresponding to a 16S ribosomal RNA gene of bacteria belonging to the genus *Geobacillus* includes an oligonucleotide comprising the nucleotide sequence of SEQ ID NO.7 below.
ACACCGAAGACCGCATGGTC (SEQ ID NO. 7)

Specific examples of oligonucleotides based on a sequence specific to the genus *Paenibacillus* among nucleotide sequences corresponding to a 16S ribosomal RNA gene of bacteria belonging to the genus *Paenibacillus* include oligonucleotides comprising a nucleotide sequence of SEQ ID NO. 3 or 8 below.
GGGCAACCTGCCTGTAAGAC (SEQ ID NO. 3)
GACGGTACCTGAGAAGAA (SEQ ID NO. 8)

In addition, other examples of capture oligonucleotides in addition to those listed above include oligonucleotides based on sequences specific to various bacteria species among base sequences corresponding to a 16S ribosomal RNA gene of *Bacillus cereus, Bacillus coagulans, Bacillus sporothermodurans, Bacillus subtilis*, *Bacillus licheniformis, Bacillus pumilus, Bacillus lentus, Bacillus firmus, Bacillus circulans, Bacillus benzoevorans, Bacillus megaterium, Bacillus sphaericus, Bacillus smithii, Bacillus fumarioli, Bacillus acidogenesis, Bacillus sonorensis, Alicyclobacillus acidoterrestris, Alicyclobacillus acidocaldarius, Alicyclobacillus hesperidum, Alicyclobacillus acadiphilus* and *Paenibacillus validus.*

Specific examples of oligonucleotides based on a sequence specific to *Bacillus cereus* among nucleotide sequences corresponding to a 16S ribosomal RNA gene *of Bacillus cereus* include oligonucleotides comprising a nucleotide sequence of SEQ ID NO.9,10 or 11 below.
GATTAAGAGCTTGCTCTTATGAA (SEQ ID NO. 9)
CCGCATGGTTCGAAAT (SEQ ID NO. 10)
GCTAGTTGAATAAGCTGGC (SEQ ID NO. 11)

Specific examples of oligonucleotides based on a sequence specific to *Bacillus coagulans* among nucleotide sequences corresponding to a 16S ribosomal RNA gene of *Bacillus coagulans* include oligonucleotides comprising a nucleotide sequence of SEQ ID NO. 12, 13 or 14 below.
TCGTGCGGACCTTTTAAAAG (SEQ ID NO. 12)
CCGCATGGAGGAAAAAG (SEQ ID NO. 13)
GCCGGGGAAGAACAAG (SEQ ID NO. 14)

Specific examples of oligonucleotides based on a sequence specific to *Bacillus sporothermodurans* among nucleotide sequences corresponding to a 16S ribosomal RNA gene of *Bacillus sporothermodurans* include oligonucleotides comprising a nucleotide sequence of SEQ ID NO. 15, 43 or 44 below.
CTCCGCATGGAGAGAGATT (SEQ ID NO. 15)
AAGAGCTTGCTTTTGATCAG (SEQ ID NO. 43)
CTTCGCATGAAGGAGAATTG (SEQ ID NO. 44)

Specific examples of oligonucleotides based on a sequence specific to *Bacillus subtilis* among nucleotide sequences corresponding to a 16S ribosomal RNA gene of *Bacillus subtilis* include oligonucleotides comprising a nucleotide sequence of SEQ ID NO. 16, 17 or 18 below.
GCATGGTTCAAACATAAAAG (SEQ ID NO. 16)
GGCTACCACTTACA (SEQ ID NO. 17)
GAGCTTGCTCCCTGATGTTA (SEQ ID NO. 18)

A specific example of an oligonucleotide based on a sequence specific to *Bacillus licheniformis* among nucleotide sequences corresponding to a 16S ribosomal RNA gene of *Bacillus licheniformis* include an oligonucleotide comprising the nucleotide sequence of SEQ ID NO. 19 below.
TGCTCCCTTAGGTCAGCGGC (SEQ ID NO. 19)

Specific examples of oligonucleotides based on a sequence specific to *Bacillus pumilus* among nucleotide sequences corresponding to a 16S ribosomal RNA gene of *Bacillus pumilus* include oligonucleotides comprising a nucleotide sequence of SEQ ID NO. 20 or 55 below.
GGATGAAAGACGGTTT (SEQ ID NO. 20)
CCGGATAGTTCCTTGAACCG (SEQ ID NO. 55)

Specific examples of oligonucleotides based on a sequence specific to *Bacillus lentus* among nucleotide sequences corresponding to a 16S ribosomal RNA gene of *Bacillus lentus* include oligonucleotides comprising a nucleotide sequence of SEQ ID NO. 21 to 25 below.
GAATGGATGGGAGCTTG (SEQ ID NO. 21)
AGCTTGCTCCCAGAAG (SEQ ID NO. 22)
TTCTCCTGGAGAAAGGTT (SEQ ID NO. 23)
AGCTTGCTCCCAGAAGTT (SEQ ID NO. 24)
CTGGAGAAAGGTTGAAAGAC (SEQ ID NO. 25)

A specific example of an oligonucleotide based on a sequence specific to *Bacillus firmus* among nucleotide sequences corresponding to a 16S ribosomal RNA gene *of Bacillus firmus* include an oligonucleotide comprising the nucleotide sequence of SEQ ID NO. 26 below.
GAGGAAAAGCTGAAAGATGG (SEQ ID NO. 26)

A specific example of an oligonucleotide based on a sequence specific to *Bacillus circulans* among nucleotide sequences corresponding to a 16S ribosomal RNA gene of *Bacillus circulans* include an oligonucleotide comprising the nucleotide sequence of SEQ ID NO. 27 below.
GAGGGGAGTTTAAAAGCTTG (SEQ ID NO. 27)

Specific examples of oligonucleotides based on a sequence specific to *Bacillus benzoevorans* among nucleotide sequences corresponding to a 16S ribosomal RNA gene of *Bacillus benzoevorans* include oligonucleotides comprising a nucleotide sequence of SEQ ID NO. 29 or 30 below.
GAGCGGACTTAAAAACGTTG (SEQ ID NO. 29)
GAGCGGACTTTTGGGAG (SEQ ID NO. 30)

A specific example of an oligonucleotide based on a sequence specific to *Bacillus megaterium* among nucleotide sequences corresponding to a 16S ribosomal RNA gene of *Bacillus megaterium* include an oligonucleotide comprising the nucleotide sequence of SEQ ID NO. 31 below.
TAGGATCTTCTCCTTCATGGG (SEQ ID NO. 31)

Specific examples of oligonucleotides based on a sequence specific to *Bacillus sphaericus* among nucleotide sequences corresponding to a 16S ribosomal RNA gene of *Bacillus sphaericus* include oliganucleotides comprising a nucleotide sequence of SEQ ID NO. 32 or 33 below.
TCGGCTGTCGCTATAGGATG (SEQ ID NO. 32)
AAGTACAGTAGTAACTGGCT (SEQ ID NO. 33)

Specific examples of oligonucleotides based on a sequence specific to *Alicyclobacillus acidoterrestris* among nucleotide sequences corresponding to a 16S ribosomal RNA gene of *Alicyclobacillus acidoterrestris* include oligonucleotides comprising a nucleotide sequence of SEQ ID NO. 34, 35 or 36 below.
TTTCAGACTGGAATAACACT (SEQ ID NO. 34)
AATACACGGGTAGGCATCTA (SEQ ID NO. 35)
GGAAAGCTCCTTGTGA (SEQ ID NO. 36)

Specific examples of oligonucleotides based on a sequence specific to *Alicyclobacillus acidocaldarius* among nucleotide sequences corresponding to a 16S ribosomal RNA gene of *Alicyclobacillus acidocaldarius* include oligonucleotides comprising a nucleotide sequence of SEQ ID NO. 28 or 37 below.
TTGGGCCGCTGAGAGAG (SEQ ID NO. 28)
CGCCCGCGAGGAGGCATCTT (SEQ ID NO. 37)

Specific examples of oligonucleotides based on a sequence specific to *Paenibacillus validus* among nucleotide sequences corresponding to a 16S ribosomal RNA gene of *Paenibacillus validus* include oligonucleotides comprising a nucleotide sequence of SEQ ID NO. 38, 39 or 40 below.
GGGGCAACCTGTGGCTTAC (SEQ ID NO. 38)
GCTAAGACCGGATAGCTGGT (SEQ ID NO. 39)
CGCCTCGGAGAGTAA (SEQ ID NO. 40)

Specific examples of oligonucleotides based on a sequence specific to *Bacillus smithii* among nucleotide sequences corresponding to a 16S ribosomal RNA gene of *Bacillus smithii* include oligonucleotides comprising a nucleotide sequence of SEQ ID NO. 45 or 46 below.
AGCTTGCTTTTTGAAAGTTA (SEQ ID NO. 45)
GATAATATCTTCCTTCGC (SEQ ID NO. 46)

Specific examples of oligonucleotides based on a sequence specific to *Alicyclobacillus acidiphilus* among nucleotide sequences corresponding to a 16S ribosomal RNA gene of *Alicyclobacillus acidiphilus* include oligonucleotides comprising a nucleotide sequence of SEQ ID NO. 47, 48 or 49 below.
GTTCAAGGGAAGGCA (SEQ ID NO. 47)
CCGTTGAGGAAAGTTGC (SEQ ID NO. 48)
ATGCAACACTGATAGAG (SEQ ID NO. 49)

Specific examples of oligonucleotides based on a sequence specific to *Alicyclobacillus hesperidum* among nucleotide sequences corresponding to a 16S ribosomal RNA gene of *Alicyclobacillus hesperidum* include oligonucleotides comprising a nucleotide sequence of SEQ ID NO. 50 or 51 below.
GGTCACGAGGAGGCA (SEQ ID NO. 50)
GCATCTTCTTGTGAGGA (SEQ ID NO. 51)

Specific examples of oligonucleotides based on a sequence specific to *Bacillus fumarioli* among nucleotide sequences corresponding to a 16S ribosomal RNA gene of *Bacillus fumarioli* include oligonucleotides comprising a nucleotide sequence of SEQ ID NO. 52 or 53 below.
TTGCCCCTTGAGATTAG (SEQ ID NO. 52)
TCATCCTTTCCTTCGC (SEQ ID NO. 53)

A specific example of an oligonucleotide based on a sequence specific to *Bacillus acidogenesis* among nucleotide sequences corresponding to a 16S ribosomal RNA gene of *Bacillus acidogenesis* includes an oligonucleotide comprising the nucleotide sequence of SEQ ID NO. 54 below.
CTTCTTCCTCCGCATGG (SEQ ID NO. 54)

Specific examples of oligonucleotides based on a sequence specific to *Bacillus sonorensis* among nucleotide sequences corresponding to a 16S ribosomal RNA gene of *Bacillus sonorensis* include oligonucleotides comprising a nucleotide sequence of SEQ ID NO. 56 or 57 below.
AGCGAACCGACGGGA (SEQ ID NO. 56)
TCCCTTAGGTTAGCGGC (SEQ ID NO. 57)

Furthermore, the above-mentioned capture oligonucleotides are not limited to oligonucleotides comprising any of the nucleotide sequences of SEQ ID NO. 1 to 40 and 43 to 57 above.

There are no particular limitations on the capture oligonucleotide immobilized on the substrate of the bacteria detection device of the present invention provided it successfully hybridizes with a probe prepared from a target bacteria. Thus, an oligonucleotide comprising any of the nucleotide sequences of SEQ ID NO. 1 to 40 and 43 to 57 may be composed only of any of the nucleotide sequences of SEQ ID NO. 1 to 40 and 43 to 57, or may comprise a sequence other than any of the nucleotide sequences of SEQ ID NO. 1 to 40 and 43 to 57. Examples of capture oligonucleotides comprising a sequence other than any of the nucleotide sequences of SEQ ID NO. 1 to 40 and 43 to 57 include oligonucleotides having a nucleotide sequence extended based on a nucleotide sequence of each 16S ribosomal RNA gene from the 5'-side, 3'-side or both of each nucleotide sequence of SEQ ID NO. 1 to 40 and 43 to 57, and a bacteria detection device in which such an oligonucleotide is immobilized on a substrate is also included in the present invention.

There are no particular limitations on the number of capture oligonucleotides immobilized on a single substrate provided at least one captured oligonucleotide is immobilized thereon. In general, in the case of detecting bacteria contaminating a sample, being able to collectively detect bacteria capable of being detected in that sample with a single substrate is preferable from the viewpoints of procedural ease and speed of testing. Thus, a bacteria detection device of the present invention is most preferably a so-called microarray type of device in which a plurality of capture oligonucleotides corresponding to a target bacteria genus or species are immobilized on a single substrate.

### [Immobilization of Oligonucleotide (Capture Oligonucleotide)]

There are no particular limitations on the method for immobilizing oligonucleotides on the substrate. A known method may be suitably selected and used. For example, a technique used in typical hybridization methods can be used, such as physical adsorption, electrical bonding or molecular covalent bonding. In a bacteria detection device of the present invention, oligonucleotides are preferably immobilized by using a base material having a carbodiimide group or a isocyanate group on the surface thereof (US Patent No. 5,908,746, Japanese Patent Application Laid-open No. H8-23975).

When spotting an oligonucleotide, if the spotted amount of the oligonucleotide is too low, it is not possible to adequately secure reactivity between the oligonucleotide and the probe, thereby making judgment difficult. In addition, high-integration spotting has technical problems while also being expensive, and a more precise, expensive detection device (such as a scanner) is required to detect hybridization signals using probe fluorescent labeling, chemical staining and so on. Thus, the oligonucleotide is preferably immobilized to a size of a diameter of 10 to 1,000 µm on the substrate surface. There are no particular limitations on the method for spotting oligonucleotides on the substrate. For example, spotting can be carried out by spotting an oligonucleotide solution on the substrate using a spotting machine. As a result thereof, the oligonucleotide solution is usually spotted in a nearly circular shape.

### (2) Bacteria Detection Method of the Present Invention

The bacteria detection method of the present invention is a method for detecting and identifying bacteria in a test sample comprising the steps of preparing a nucleic acid of bacteria in a test sample, preparing a labeled probe by using the nucleic acid as a template, hybridizing the labeled probe with an oligonucleotide based on a sequence specific to the species or genus to which the target bacteria belongs, and detecting a hybridization signal. The above-mentioned bacteria detection device of the present invention is preferably used in the hybridization step of this detection method. The use of this bacteria detection device makes it possible to easily, rapidly, accurately and comprehensively detect and identify target bacteria. In addition, a food is preferable for the test sample of this detection method, while a beverage is more preferable, and a refreshing beverage is the most preferable. The following provides a detailed explanation of each step of this detection method.

### [Nucleic Acid Preparation Step]

In this step, a nucleic acid is prepared from bacteria in the test sample. A known nucleic acid preparation method can be suitably selected and used for the method for preparing a nucleic acid from the test sample. For example, DNA can be prepared by extracting in accordance with the method introduced by R.F. Wang (Molecular and Cellular Probes (2000), 14, 1-5). Although this is a standard preparation method, it goes without saying that numerous alternative methods may also be used. In addition, a cormnercially available kit may also be used.

### [Probe Preparation Step]

In this step, a labeled probe is prepared by using the nucleic acid prepared in the above-mentioned nucleic acid preparation step as a template. The probe can be prepared by nucleic acid amplification using primers designed so as to amplify regions comprising the nucleotide sequences of a capture oligonucleotide and a positive control capture oligonucleotide. Examples of methods for amplifying nucleic acids include, but are not limited to, amplification in the form of DNA by PCR or amplification as RNA by in vitro transcription.

For example, in the case of preparing a labeled probe by PCR, the primers used in PCR are designed so as to amplify regions comprising nucleotide sequences complementary to a capture oligonucleotide and a positive control capture oligonucleotide. Furthermore, the probe may be longer than the capture oligonucleotide or positive control capture oligonucleotide provided hybridization is still possible. The primers used in PCR can be labeled in advance to obtain a labeled probe. In addition, a labeled probe can also be obtained by labeling the PCR substrate (deoxynucleoside triphosphate). Alternatively, the probe may be labeled following completion of PCR. There are no particular limitations on the label. Examples of labels that can be used include fluorescent substances, haptens, radioactive substances and other labels similar to the probe ordinarily used in hybridization. Specific examples of fluorescent substances include fluorescein (FITC), rodamine, phecoerythrin (PE), Texas Red and cyanine-based fluorescent dyes, while specific examples of haptens include biotin, digoxigenin (Dig), dinitrophenyl (DNP) and fluorescein.

### [Hybridization Step]

In the hybridization step, the labeled probe is hybridized with an oligonucleotide based on a sequence specific to the species or genus of the target bacteria. Although hybridization can be carried out on a membrane on which the above-mentioned oligonucleotide is immobilized, the bacteria detection device of the present invention is used preferably. The use of this bacteria detection device makes it possible to easily, rapidly, accurately and comprehensively detect and identify target bacteria. There are no particular limitations on the method used in the hybridization step, and a known nucleic acid hybridization method can be suitably selected and used. The following indicates a specific example of a hybridization method.

A labeled probe is added to a fusion solution comprised of a salt solution such as standard saline citrate (SSC), a blocking solution such as sodium dodecyl sulfate (SDS) or bovine serum albumin (BSA), and an additive for promoting a fusion reaction. In the case the probe is double stranded, it is denatured with heat and so on. After adding several microliters of labeled probe solution to the substrate, a heating procedure is carried out for several hours (normally at 37 to 50°C) to form a hybrid between labeled probe and oligonucleotide immobilized on the substrate. Subsequently, 5×SSC or 3 M tetramethylammonium chloride is added to the substrate followed by heating (normally at 37 to 50°C) to release labeled probe which has not formed a specific hybrid from the substrate and selectively leave only a specific hybrid on the substrate.

### [Signal Detection Step]

In this step, a judgment is made as to the presence or absence of successful hybridization in the above-mentioned hybridization step. This step is normally carried out continuously from the hybridization step.

The method used in the signal detection step depends on the label introduced to the probe prepared in the above-mentioned probe preparation step. Namely, a label such as a fluorescent substance or hapten introduced to the probe is used for detecting a hybrid. Thus, a known method for detecting the label introduced to the probe used may be suitably selected and used.

In the case of using a hapten, for example, a solution containing a conjugate of a protein that recognizes the hapten or binds thereto, and alkaline phosphatase or horseradish peroxidase and so on, is added to the substrate followed by reacting for several tens of minutes at room temperature.

The hybrid is visualized by adding a compound so as to form an insoluble compound only in the case a hapten and enzyme conjugate are present. The formation of this insoluble compound is visualized as a result of being amplified by an enzymatic reaction. Examples of added compounds include nitro blue tetrazolium chloride (NBT) and BCIP (5-bromo-4-chloro-3-indolyl phosphate-p-toluidine salt) in the case the enzyme present in the enzyme conjugate is alkaline phosphatase, and TMB (3,3',5,5'-tetramethyl benzidine) in the case the enzyme is horseradish peroxidase.

A bacteria species contained in a test sample can be determined by viewing a hybridization signal such as pigment deposition or fluorescence emission of a hybrid at the location where the capture oligonucleotide is immobilized. Namely, in the case a hybridization signal has been detected, it means that bacteria corresponding to an oligonucleotide spotted at the location of the signal is contained in the test sample. Furthermore, if a signal is observed at the location of the positive control capture oligonucleotide, the test can be confirmed to be functioning properly. If a signal is observed at the location of the negative control capture oligonucleotide, the hybridization can be confirmed to have been carried out under suitable conditions.

### (3) Bacteria Detection Kit of the Present Invention

The bacteria detection kit of the present invention is a kit for carrying out the bacteria detection method of the present invention as described above. Thus, there are no particular limitations on the components contained in the kit provided it allows the bacteria detection method of the present invention to be carried out.

This bacteria detection kit preferably comprises the bacteria detection device of the present invention. The use of a kit comprising this bacteria detection device makes it possible to easily, rapidly, accurately and comprehensively detect and identify target bacteria. In addition, this bacteria detection kit preferably also comprises reagents used in the above-mentioned hybridization step and signal detection step. Examples of reagents used in the hybridization step include a salt solution such as standard saline citrate (SSC), a blocking solution such as sodium dodecyl sulfate (SDS) or bovine serum albumin (BSA), and an additive for promoting a fusion reaction.

In addition, examples of reagents used in the signal detection step include conjugates of an enzyme and a protein that recognizes a hapten (enzyme conjugates), and chromogenic substrates such as NBT, BCIP or TMB.

Furthermore, the reagents used in the hybridization step and signal detection step are not limited to the previously listed examples, but rather the kit may be composed by selecting suitable and appropriate reagents to match the purpose of use.

The present bacteria detection kit preferably comprises a reagent used in the above-mentioned probe preparation step, and more preferably also comprises a reagent used in the above-mentioned nucleic acid preparation step. Examples of reagents used in the probe preparation step include PCR buffers, heat-resistant DNA synthases and mixtures of deoxynucleotide triphosphates, while examples of reagents used in the nucleic acid preparation step include lysis buffers, DNA recovery columns and DNA extraction buffers. However, these reagents are not limited to the examples listed above, but rather the kit may be composed by selecting suitable and appropriate reagents to match the purpose of use.

The use of the kit of the present invention (comprising the bacteria detection device of the present invention and reagents used in each step) makes it possible to carry out detection and identification of bacteria contained in a test sample in about 6 hours after receiving the test sample.

### (4) Applications of the Present Invention

There are no particular limitations on the applications of the bacteria detection device, bacteria detection method and bacteria detection kit of the present invention, and they can be used in all applications requiring determination of bacteria. More specifically, they can be preferably used in cases requiring rapid and accurate detection and identification of bacteria isolated from industrial products or the production environments in the production processes of various types of industrial products for which bacterial contamination has a considerable effect on product quality.

Typical examples of the above-mentioned industrial products serving as a source of bacteria to be subjected to determination include, but are not limited to, foods, pharmaceuticals, reagents, over-the-counter medicines and disposable medical devices. Among the industrial products listed above, the present invention is preferably applied to foods. Specific examples of foods include, but are not limited to, beverages (refreshing beverages, alcoholic beverages, concentrated reconstituted fruit juices, natural fruit juices, fruit beverages, coffee drinks), breads, confections (including frozen confections), cooked foods, dairy products, cereals, bean curd and fried bean curd, noodles, box lunches, condiments, wheat flour, meats and other agricultural products, nutritional supplements and long-term storage foods (such as canned goods, frozen foods and pre-cooked foods).

Among the examples of foods described above, the present invention can be preferably applied to refreshing beverages in particular. Examples of refreshing beverages include, but are not limited to teas such as barley tea, Oolong tea, green tea and kazusa leaf tea.

While preferred embodiments of the invention have been described and illustrated above, it should be understood that these are exemplary of the invention and are not to be considered as limiting. Various modifications can be made without departing from the spirit or scope of the present invention as of the claims, and embodiments obtained by suitably combining technical means respectively disclosed in different embodiments are also included in the technical scope of the present invention.

### EXAMPLES

### [Oligonucleotide Synthesis]

Oligonucleotides were synthesized using an oligonucleotide synthesizer (Perkin-Elmer Applied Biosystems) in accordance with established methods followed by deprotection and drying. The dried oligonucleotides were dissolved using 10 mM Tris-HCl (pH 7.5) and 1 mM EDTA buffer to prepare a 100 pmol/µl oligonucleotide solution. All of the oligonucleotides used in the examples/embodiments were synthesized by this method.

The nucleotide sequences of the synthesized oligonucleotides are shown in SEQ ID NO. 1 to 57. Among these, SEQ ID NO. 1 to 40 and 43 to 57 are capture oligonucleotides, while SEQ ID NO. 41 and 42 are primers. An amino group was coupled to the 5'-end of the capture oligonucleotides, while biotin was coupled to the 5'-end of the primers using the synthesizer described above.

### [Spotting of Capture Oligonucleotides onto Substrate]

10 µl of microspotting solution (TeleChem International) were mixed with 10 µl of the solution containing oligonucleotide having an amino group on the 5'-end thereof followed by apportioning into the wells of a microtiter plate (Greiner Laboratory). A carbodiimide resin-treated slide glass (Carbostation, Nisshinbo Industries, registered trademark) was arranged at predetermined location on the spotting machine followed by operation of the spotting machine.

Following completion of spotting, steam from hot water was applied to the slide glass for several seconds followed by irradiating with 600 mJ of ultraviolet light. After again exposing the slide glass to steam for several seconds, the slide glass was placed on a hot plate to remove moisture.

The slide glass was then immersed in a mixture of 100 mM Tris-HCl (pH 7.5), 100 mM NaCl and 0.1% Triton X-100 containing 3% bovine serum albumin (BSA) for 30 minutes at room temperature to carry out blocking. Subsequently, the slide glass was washed with 10 mM Tris-HCl (pH 7.5) and 1 mM EDTA buffer.

The slide glass was dried at room temperature and stored in a cool, dark location in a dry state until the time of use.

### [Nucleic Acid Preparation Step]

*Bacillus* species used as specimens consisted of *Bacillus cereus, Bacillus coagulans, Bacillus sporothermodurans, Bacillus subtilis, Bacillus licheniformis, Bacillus pumilus, Bacillus lentus, Bacillus firmus, Bacillus circulans, Bacillus benzoevorans, Bacillus megaterium, Bacillus sphaericus, Bacillus smithii, Bacillus fumarioli, Bacillus acidogenesis* and *Bacillus sonorensis,* which belong to the genus *Bacillus.*

*Alicyclobacillus* species used as specimens consisted of *Alicyclobacillus acidoterrestris, Alicyclobacillus acidocaldarius, Alicyclobacillus acidiphilus* and *Alicyclobacillus hesperidum,* which belong to the genus *Alicyclobacillus.*

*Paenibacillus* species used as specimens consisted of *Paenibacillus validus, Paenibacillus illinoisensis, Paenibacillus alvei* and *Paenibacillus polymyxa,* which belong to the genus *Paenibacillus..*

*Aneurinibacillus* species used as specimens consisted of *Aneurinibacillus aneurinolyticus,* which belong to the genus *Aneurinibacillus.*

*Geobacillus* species used as specimens consisted of *Geobacillus stearothermophilus* which belong to the genus *Geobacillus.*

*Brevibacillus* species used as specimens consisted of *Brevibacillus brevis,* which belong to the genus *Brevibacillus.*

On the other hand, control specimens used consisted of *Escherichia coli, Staphylococcus aureus, Staphylococcus capitis, Streptococcus salivarius, Pseudomonas putida* and *Lactobacillus delbrueckii.*

Genomic DNA of each species were respectively prepared from cultures of each organism cultured under optimum conditions using the Genomic DNA Purification Kit (Edge Biosystems, Cat. No. 85171).

### [Probe Preparation Step]

A probe nucleic acid was prepared by PCR using the resulting DNA of each bacteria as a template. The composition of the PCR reaction solution consisted of the addition of sterilized distilled water to 2.5 units of Taq polymerase, 50 pmol each of biotinated primers (SEQ ID NO. 41 and 42), 5 µl of 10× reaction buffer, 10 nmol each of dNTP and 100 ng of template DNA to bring to a final volume of 50 µl. After holding for 3 minutes at 95°C, 40 cycles of a reaction consisting of 30 seconds at 95°C, 30 seconds at 57°C and 1 minute at 72°C were carried out, followed by holding for 5 minutes at 72°C to complete the reaction.

### [Hybridization Step and Signal Detection Step]

Four µL of the probe nucleic acid solution and 16 µL of Arraylt Unihyb Hybridization Solution (TeleChem International) were combined and mixed followed by heat treatment for 1 minute at 95°C and immersing in ice for 1 minute. The entire amount of this probe nucleic acid solution was then placed on a capture oligonucleotide-immobilized substrate, and a cover glass was placed thereon. This was placed in a moisturizing chamber and allowed to stand undisturbed for 120 minutes in a constant humidity incubator set to 37°C. The substrate was then removed and promptly immersed in 2×SSC solution (2×SSC: 0.033 M NaCl, 0.033 M sodium citrate) at room temperature followed by removing the cover glass and immersing for 5 minutes in the 2×SSC solution warmed to 37°C.

The substrate was removed from the 2×SSC solution, placed in a centrifuge (Beckman) and centrifuged for 1 minute at 2,000 rpm. Next, an avidin-biotinated peroxidase conjugate was prepared using the Vectastain Elite ABC Kit (Vector), and 1.4 ml were dropped onto the substrate followed by allowing to stand undisturbed for 30 minutes at room temperature.

Subsequently, the substrate was washed in PBS (10 mM sodium phosphate (pH 7.5), 0.9% sodium chloride). A chromogenic solution was prepared using the TMB Substrate Kit for Peroxidase (Vector) and 1.4 ml of this solution were dropped onto the substrate followed by allowing to stand undisturbed for 30 minutes at room temperature. The substrate was then washed with distilled water to stop the chromogenic reaction.

### [Determination]

The region where hybridization was carried out was scanned at 600 dpi using an Epson Scanner Model GT-8700F and the transmission unit provided therewith, and the scanned images were visually checked for the presence of color generation.

### [Results]

The results for the 21 species of bacteria used as specimens are shown in Tables 1 to 21.

Furthermore, in the tables, a "+" indicates a spot for which coloring was confirmed, while a "-" indicates a spot for which coloring was not confirmed.

The results for *Bacillus subtilis* are shown in Table 1.

**Table 1 Test Bacteria: Bacillus subtilis**

| Target Bacteria | SEQ ID NO./+ (signal present), - (signal absent) | | | | |
|---|---|---|---|---|---|
| Genus *Bacillus* | 1- | 2+ | 3+ | | |
| Genus *Brevibactillus* | 4- | | | | |
| Genus *Alicyclobacillus* | 5- | | | | |
| Genus *Aneurinibacillus* | 6- | | | | |
| Genus *Geobacillus* | 7- | | | | |
| Genus *Paenibacillus* | 8- | | | | |
| *Bacillus cereus* | 9- | 10- | 11- | | |
| *Bacillus coagulans* | 12- | 13- | 14- | | |
| *Bacillus sporothermodurans* | 15- | 43- | 44- | | |
| *Bacillus subtilis* | 16+ | 17+ | 18+ | | |
| *Bacillus licheniformis* | 19- | | | | |
| *Bacillus pumilus* | 20- | 55- | | | |
| *Bacillus lentus* | 21- | 22- | 23- | 24- | 25- |
| *Bacillus firmus* | 26- | | | | |
| *Bacillus circulans* | 27- | | | | |
| *Baclllus benzoevorans* | 29- | 30- | | | |
| *Bacillus megaterium* | 31- | | | | |
| *Bacillus sphaericus* | 32- | 33- | | | |
| *Alicyclobacillus acidoterrestris* | 34- | 35- | 36- | | |
| *Alicyclobacillus acidocaldarius* | 28- | 37- | | | |
| *Paenibacillus validus* | 38- | 39- | 40- | | |
| *Bacillus smithii* | 45- | 46- | | | |
| *Alicyclobacillus acidiphilus* | 47- | 48- | 49- | | |
| *Alicyclobacillus hesperidum* | 50- | 51- | | | |
| *Bacillus fumarioli* | 52- | 53- | | | |
| *Bacillus acidogenesis* | 54- | | | | |
| *Bacillus sonorensis* | 56- | 57- | | | |

According to Table 1, color generation was confirmed for *Bacillus subtilis* at a total of five locations of capture oligonucleotides for detection of the genus *Bacillus* (detection of bacteria belonging to the genus *Bacillus)* (SEQ ID NO. 2 and 3) and capture oligonucleotides for detection of *Bacillus subtilis* (SEQ ID NO. 16, 17 and 18).

Table 2 shows the results for *Bacillus coagulans.*

**Table 2 Test Bacteria: Bacillus coagulans**

| Target Bacteria | SEQ ID NO./+ (signal present), - (signal absent) | | | | |
|---|---|---|---|---|---|
| Genus *Bacillus* | 1+ | 2- | 3+ | | |
| Genus *Brevibacillus* | 4- | | | | |
| Genus *Alicyclobacillus* | 5- | | | | |
| Genus *Aneurinibacillus* | 6- | | | | |
| Genus *Geobacillus* | 7- | | | | |
| Genus *Paenibacillus* | 8- | | | | |
| *Bacillus cereus* | 9- | 10- | 11- | | |
| *Bacillus coagulans* | 12+ | 13+ | 14+ | | |
| *Bacillus sporothermodurans* | 15- | 43- | 44- | | |
| *Bacillus subtilis* | 16- | 17- | 18- | | |
| *Bacillus licheniformis* | 19- | | | | |
| *Bacillus pumilus* | 20- | 55- | | | |
| *Bacillus lentus* | 21- | 22- | 23- | 24- | 25- |
| *Bacillus firmus* | 26- | | | | |
| *Bacillus circulans* | 27- | | | | |
| *Bacillus benzoevorans* | 29- | 30- | | | |
| *Bacillus megaterium* | 31- | | | | |
| *Bacillus sphaericus* | 32- | 33- | | | |
| *Alicyclobacillus acidoterrestris* | 34- | 35- | 36- | | |
| *Alicyclobacillus acidocaldarius* | 28- | 37- | | | |
| *Paenibacillus validus* | 38- | 39- | 40- | | |
| *Bacillus smithii* | 45- | 46- | | | |
| *Alicyclobacillus acidiphilus* | 47- | 48- | 49- | | |
| *Alicyclobacillus hesperidum* | 50- | 51- | | | |
| *Bacillus fumarioli* | 52- | 53- | | | |
| *Bacillus acidogenesis* | 54- | | | | |
| *Bacillus sonorensis* | 56- | 57- | | | |

According to Table 2, color generation was confirmed for *Bacillus coagulans* at a total of five locations of capture oligonucleotides for detection of the genus *Bacillus* (SEQ ID NO. 1 and 3) and capture oligonucleotides for detection of *Bacillus coagulans* (SEQ ID NO. 12, 13 and 14).

Table 3 shows the results for *Bacillus sphaericus.*

**Table 3 Test Bacteria: Bacillus sphaericus**

| Target Bacteria | SEQ ID NO./+ (signal present), - (signal absent) | | | | |
|---|---|---|---|---|---|
| Genus *Bacillus* | 1+ | 2- | 3- | | |
| Genus *Brevibacillus* | 4- | | | | |
| Genus *Alicyclobacillus* | 5- | | | | |
| Genus *Aneurinibacillus* | 6- | | | | |
| Genus *Geobacillus* | 7- | | | | |
| Genus *Paenibacillus* | 8- | | | | |
| *Bacillus cereus* | 9- | 10- | 11- | | |
| *Bacillus coagulans* | 12- | 13- | 14- | | |
| *Bacillus sporothermodurans* | 15- | 43- | 44- | | |
| *Bacillus subtilis* | 16- | 17- | 18- | | |
| *Bacillus licheniformis* | 19- | | | | |
| *Bacillus pumilus* | 20- | 55- | | | |
| *Bacillus lentus* | 21- | 22- | 23- | 24- | 25- |
| *Bacillus firmus* | 26- | | | | |
| *Bacillus circulans* | 27- | | | | |
| *Bacillus benzoevorans* | 29- | 30- | | | |
| *Bacillus megaterium* | 31- | | | | |
| *Bacillus sphaericus* | 32+ | 33+ | | | |
| *Alicyclobacillus acidolerrestris* | 34- | 35- | 36- | | |
| *Alicyclobacillus acidocaldarius* | 28- | 37- | | | |
| *Paenibacillus validus* | 38- | 39- | 40- | | |
| *Bacillus smithii* | 45- | 46- | | | |
| *Alicyclobacillus acidiphilus* | 47- | 48- | 49- | | |
| *Alicyclobacillus hesperidum* | 50- | 51- | | | |
| *Bacillus fumarioli* | 52- | 53- | | | |
| *Bacillus acidogenesis* | 54- | | | | |
| *Bacillus sonorensis* | 56- | 57- | | | |

According to Table 3, color generation was confirmed for *Bacillus sphaericus* at a total of three locations of a capture oligonucleotide for detection of the genus *Bacillus* (SEQ ID NO. 1) and capture oligonucleotides for detection of *Bacillus sphaericus* (SEQ ID NO. 32 and 33).

Table 4 shows the results for *Bacillus pumilus.*

**Table 4 Test Bacteria: Bacillus pumilus**

| Target Bacteria | SEQ ID NO./+ (signal present), - (signal absent) | | | | |
|---|---|---|---|---|---|
| Genus *Bacillus* | 1- | 2- | 3+ | | |
| Genus *Brevibacillus* | 4- | | | | |
| Genus *Alicyclobacillus* | 5- | | | | |
| Genus *Aneurinibacillus* | 6- | | | | |
| Genus *Geobacillus* | 7- | | | | |
| Genus *Paerabacillus* | 8- | | | | |
| *Bacillus cereus* | 9- | 10- | 11- | | |
| *Bacillus coagulans* | 12- | 13- | 14- | | |
| *Bacillus sporothermodurans* | 15- | 43- | 44- | | |
| *Bacillus subtilis* | 16- | 17- | 18- | | |
| *Bacillus licheniformis* | 19- | | | | |
| *Bacillus pumilus* | 20+ | 55+ | | | |
| *Bacillus lentus* | 21- | 22- | 23- | 24- | 25- |
| *Bacillus firmus* | 26- | | | | |
| *Bacillus circulans* | 27- | | | | |
| *Bacillus benzoevorans* | 29- | 30- | | | |
| *Bacillus megaterium* | 31- | | | | |
| *Bacillus sphaericus* | 32- | 33- | | | |
| *Alicyclobacillus acidoterrestris* | 34- | 35- | 36- | | |
| *Alicyclobacillus acidocaldarius* | 28- | 37- | | | |
| *Paenibacillus validus* | 38- | 39- | 40- | | |
| *Bacillus smithii* | 45- | 46- | | | |
| *Alicyclobacillus acidiphilus* | 47- | 48- | 49- | | |
| *Alicyclobacillus hesperidum* | 50- | 51- | | | |
| *Bacillus fumarioli* | 52- | 53- | | | |
| *Bacillus acidogenesis* | 54- | | | | |
| *Bacillus sonorensis* | 56- | 57- | | | |

According to Table 4, color generation was confirmed for *Bacillus pumilus* at a total of three locations of a capture oligonucleotide for detection of the genus *Bacillus* (SEQ ID NO. 3) and capture oligonucleotides for detection of *Bacillus pumilus* (SEQ ID NO. 20 and 55).

Table 5 shows the results for *Bacillus sporothermodurans.*

**Table 5 Test Bacteria: Bacillus sporothermodurans**

| Target Bacteria | SEQ ID NO./+ (signal present), - (signal absent) | | | | |
|---|---|---|---|---|---|
| Genus *Bacillus* | 1- | 2- | 3+ | | |
| Genus *Brevibcillus* | 4- | | | | |
| Genus *Alicyclobacillus* | 5- | | | | |
| Genus *Aneurinibacillus* | 6- | | | | |
| Genus *Geobacillus* | 7- | | | | |
| Genus *Paenibacillus* | 8- | | | | |
| *Bacillus cereus* | 9- | 10- | 11- | | |
| *Bacillus coagulans* | 12- | 13- | 14- | | |
| *Bacillus sporothermodurans* | 15+ | 43+ | 44- | | |
| *Bacillus subtilis* | 16- | 17- | 18- | | |
| *Bacillus licheniformis* | 19- | | | | |
| *Bacillus pumilus* | 20- | 55- | | | |
| *Bacillus lentus* | 21- | 22- | 23- | 24- | 25- |
| *Bacillus firmus* | 26- | | | | |
| *Bacillus circulans* | 27- | | | | |
| *Bacillus benzoevorans* | 29- | 30- | | | |
| *Bacillus megaterium* | 31- | | | | |
| *Bacillus sphaericus* | 32- | 33- | | | |
| *Alicyclobacillus acidoterrestris* | 34- | 35- | 36- | | |
| *Alicyclobacillus acidocaidarius* | 28- | 37- | | | |
| *Paenibacillus validus* | 38- | 39- | 40- | | |
| *Bacillus smithii* | 45- | 46- | | | |
| *Alicyclobacillus acidiphilus* | 47- | 48- | 49- | | |
| *Alicyclobacillus hesperidum* | 50- | 51- | | | |
| *Bacillus fumarioli* | 52- | 53- | | | |
| *Bacillus acidogenesis* | 54- | | | | |
| *Bacillus sonorensis* | 56- | 57- | | | |

According to Table 5, color generation was confirmed for *Bacillus sporothermodurans* at a total of three locations of a capture oligonucleotides for detection of the genus *Bacillus* (SEQ ID NO. 3) and capture oligonucleotides for detection of *Bacillus sporothermodurans* (SEQ ID NO. 15 and 43).

Table 6 shows the results for *Bacillus megaterium.*

**Table 6 Test Bacteria: Bacillus megaterium**

| Target Bacteria | SEQ ID NO./+ (signal present), - (signal absent) | | | | |
|---|---|---|---|---|---|
| Genus *Bacillus* | 1+ | 2- | 3+ | | |
| Genus *Brevibacillus* | 4- | | | | |
| Genus *Alicyclobacillus* | 5- | | | | |
| Genus *Aneurinibacillus* | 6- | | | | |
| Genus *Geobacillus* | 7- | | | | |
| Genus *Paerabacillus* | 8- | | | | |
| *Bacillus cereus* | 9- | 10- | 11- | | |
| *Bacillus coagulans* | 12- | 13- | 14- | | |
| *Bacillus sporothermodurans* | 15- | 43- | 44- | | |
| *Bacillus subtilis* | 16- | 17- | 18- | | |
| *Bacillus licheniformis* | 19- | | | | |
| *Bacillus pumilus* | 20- | 55- | | | |
| *Bacillus lentus* | 21- | 22- | 23- | 24- | 25- |
| *Bacillus firmus* | 26- | | | | |
| *Bacillus circulans* | 27- | | | | |
| *Bacillus benzoevorans* | 29- | 30- | | | |
| *Bacillus megaterium* | 31+ | | | | |
| *Bacillus sphaericus* | 32- | 33- | | | |
| *Alicyclobacillus acidoterrestris* | 34- | 35- | 36- | | |
| *Alicyclobacillus acidocaldarius* | 28- | 37- | | | |
| *Paenibacillus validus* | 38- | 39- | 40- | | |
| *Bacillus smithii* | 45- | 46- | | | |
| *Alicyclobacillus acidiphilus* | 47- | 48- | 49- | | |
| *Alicyclobacillus hesperidum* | 50- | 51- | | | |
| *Bacillus fumarioli* | 52- | 53- | | | |
| *Bacillus acidogenesis* | 54- | | | | |
| *Bacillus sonorensis* | 56- | 57- | | | |

According to Table 6, color generation was confirmed for *Bacillus megaterium* at a total of three locations of capture oligonucleotides for detection of the genus *Bacillus* (SEQ ID NO. 1 and 3) and a capture oligonucleotide for detection of *Bacillus megaterium* (SEQ ID NO.31).

Table 7 shows the results for *Bacillus lentus.*

**Table 7 Test Bacteria: Bacillus lentus**

| Target Bacteria | SEQ ID NO./+ (signal present), - (signal absent) | | | | |
|---|---|---|---|---|---|
| Genus *Bacillus* | 1+ | 2- | 3+ | | |
| Genus *Brevibacillus* | 4- | | | | |
| Genus *Alicyclob*a*cillus* | 5- | | | | |
| Genus *Aneurinibacillus* | 6- | | | | |
| Genus *Geobacillus* | 7- | | | | |
| Genus *Paenibacillus* | 8- | | | | |
| *Bacillus cereus* | 9- | 10- | 11- | | |
| *Bacillus coagulans* | 12- | 13- | 14- | | |
| *Bacillus sporothermodurans* | 15- | 43- | 44- | | |
| *Bacillus subtilis* | 16- | 17- | 18- | | |
| *Bacillus licheniformis* | 19- | | | | |
| *Bacillus pumilus* | 20- | 55- | | | |
| *Bacillus lentus* | 21+ | 22+ | 23+ | 24+ | 25+ |
| *Bacillus firmus* | 26- | | | | |
| *Bacillus circulans* | 27- | | | | |
| *Bacillus benzoevorans* | 29- | 30- | | | |
| *Bacillus megaterium* | 31- | | | | |
| *Bacillus sphaericus* | 32- | 33- | | | |
| *Alicyclobacillus acidoterrestris* | 34- | 35- | 36- | | |
| *Alicyclobacillus acidocaldarius* | 28- | 37- | | | |
| *Paenibacillus validus* | 38- | 39- | 40- | | |
| *Bacillus smithii* | 45- | 46- | | | |
| *Alicyclobacillus acidiphilus* | 47- | 48- | 49- | | |
| *Alicyclobacillus hesperidum* | 50- | 51- | | | |
| *Bacillus fumarioli* | 52- | 53- | | | |
| *Bacillus acidogenesis* | 54- | | | | |
| *Bacillus sonorensis* | 56- | 57- | | | |

According to Table 7, color generation was confirmed for *Bacillus lentus* at a total of seven locations of capture oligonucleotides for detection of the genus *Bacillus* (SEQ ID NO. 1 and 3) and capture oligonucleotides for detection of *Bacillus lentus* (SEQ ID NO. 21, 22, 23, 24 and 25),

Table 8 shows the results for *Bacillus firmus.*

**Table 8 Test Bacteria: Bacillus firmus**

| Target Bacteria | SEQ ID NO./+ (signal present), - (signal absent) | | | | |
|---|---|---|---|---|---|
| Genus *Bacillus* | 1+ | 2- | 3+ | | |
| Genus *Brevibacillus* | 4- | | | | |
| Genus *Alicyclobacillus* | 5- | | | | |
| Genus *Aneurinibacillus* | 6- | | | | |
| Genus *Geobacillus* | 7- | | | | |
| Genus *Paenibacillus* | 8- | | | | |
| *Bacillus cereus* | 9- | 10- | 11- | | |
| *Bacillus coagulans* | 12- | 13- | 14- | | |
| *Bacillus sporothermodurans* | 15- | 43- | 44- | | |
| *Bacillus subtilis* | 16- | 17- | 18- | | |
| *Bacillus licheniformis* | 19- | | | | |
| *Bacillus pumilus* | 20- | 55- | | | |
| *Bacillus lentus* | 21- | 22- | 23- | 24- | 25- |
| *Bacillus firmus* | 26+ | | | | |
| *Bacillus circulans* | 27- | | | | |
| *Bacillus benzoevorans* | 29- | 30- | | | |
| *Bacillus megaterium* | 31- | | | | |
| *Bacillus sphaericus* | 32- | 33- | | | |
| *Alicyclobacillus acidoterrestris* | 34- | 35- | 36- | | |
| *Alicyclobacillus acidocaldarius* | 28- | 37- | | | |
| *Paenibacillus validus* | 38- | 39- | 40- | | |
| *Bacillus smithii* | 45- | 46- | | | |
| *Alicyclobacillus acidiphilus* | 47- | 48- | 49- | | |
| *Alicyclobacillus hesperidum* | 50- | 51- | | | |
| *Bacillus fumarioli* | 52- | 53- | | | |
| *Bacillus acidogenesis* | 54- | | | | |
| *Bacillus sonorensis* | 56- | 57- | | | |

According to Table 8, color generation was confirmed for *Bacillus firmus* at a total of three locations of capture oligonucleotides for detection of the genus *Bacillus* (SEQ ID NO. 1 and 3) and a capture oligonucleotide for detection of *Bacillus firmus* (SEQ ID NO. 26).

Table 9 shows the results for *Bacillus circulans.*

**Table 9 Test Bacteria: Bacillus circulans**

| Target Bacteria | SEQ ID NO./+ (signal present), - (signal absent) | | | | |
|---|---|---|---|---|---|
| Genus *Bacillus* | 1+ | 2- | 3+ | | |
| Genus *Brevibacillus* | 4- | | | | |
| Genus *Alicyclobacillus* | 5- | | | | |
| Genus *Aneurinibacillus* | 6- | | | | |
| Genus *Geobacillus* | 7- | | | | |
| Genus *Paenibacillus* | 8- | | | | |
| *Bacillus cereus* | 9- | 10- | 11- | | |
| *Bacillus coagulans* | 12- | 13- | 14- | | |
| *Bacillus sporothermodurans* | 15- | 43- | 44- | | |
| *Bacillus subtilis* | 16- | 17- | 18- | | |
| *Bacillus licheniformis* | 19- | | | | |
| *Bacillus pumilus* | 20- | 55- | | | |
| *Bacillus lentus* | 21- | 22- | 23- | 24- | 25- |
| *Bacillus firmus* | 26- | | | | |
| *Bacillus circulans* | 27+ | | | | |
| *Bacillus benzoevorans* | 29- | 30- | | | |
| *Bacillus megaterium* | 31- | | | | |
| *Bacillus sphaericus* | 32- | 33- | | | |
| *Alicyclobaci*/*lus acidoterrestris* | 34- | 35- | 36- | | |
| *Alicyclobacillus acidocaldarius* | 28- | 37- | | | |
| *Poenibacillus validus* | 38- | 39- | 40- | | |
| *Bacillus smithii* | 45- | 46- | | | |
| *Alicyclobacillus acidiphilus* | 47- | 48- | 49- | | |
| *Alicyc*/*obacillus hesperidum* | 50- | 51- | | | |
| *Bacillus fumarioli* | 52- | 53- | | | |
| *Bacillus acidogenesis* | 54- | | | | |
| *Bacillus sonorensis* | 56- | 57- | | | |

According to Table 9, color generation was confirmed for *Bacillus circulans* at a total of three locations of capture oligonucleotides for detection of the genus *Bacillus* (SEQ ID NO. 1 and 3) and a capture oligonucleotide for detection of *Bacillus circulans* (SEQ ID NO. 27).

Table 10 shows the results for *Bacillus benzoevorans.*

**Table 10 Test Bacteria: Bacillus benzoevorans**

| Target Bacteria | SEQ ID NO./+ (signal present), - (signal absent) | | | | |
|---|---|---|---|---|---|
| Genus *Bacillus* | 1+ | 2- | 3+ | | |
| Genus *Brevibacillus* | 4- | | | | |
| Genus *Alicyclobacillus* | 5- | | | | |
| Genus *Aneurinibacillus* | 6- | | | | |
| Genus *Geobacillus* | 7- | | | | |
| Genus *Paenibacillus* | 8- | | | | |
| *Bacillus cereus* | 9- | 10- | 11- | | |
| *Bacillus coagulans* | 12- | 13- | 14- | | |
| *Bacillus sporothermodurans* | 15- | 43- | 44- | | |
| *Bacillus subtilis* | 16- | 17- | 18- | | |
| *Bacillus licheniformis* | 19- | | | | |
| *Bacillus pumilus* | 20- | 55- | | | |
| *Bacillus lentus* | 21- | 22- | 23- | 24- | 25- |
| *Bacillus firmus* | 26- | | | | |
| *Bacillus circulans* | 27- | | | | |
| *Bacillus benzoevorans* | 29- | 30+ | | | |
| *Bacillus megaterium* | 31- | | | | |
| *Bacillus sphaericus* | 32- | 33- | | | |
| *Alicyclobacillus acidoterrestris* | 34- | 35- | 36- | | |
| *Alicyclobacillus acidocaldarius* | 28- | 37- | | | |
| *Paenibacillus validus* | 38- | 39- | 40- | | |
| *Bacillus smithii* | 45- | 46- | | | |
| *Alicyclobacillus acidiphilus* | 47- | 48- | 49- | | |
| *Alicyclobacillus hesperidum* | 50- | 51- | | | |
| *Bacillus fumarioli* | 52- | 53- | | | |
| *Bacillus acidogenesis* | 54- | | | | |
| *Bacillus sonorensis* | 56- | 57- | | | |

According to Table 10, color generation was confirmed for *Bacillus benzoevorans* at a total of three locations of capture oligonucleotides for detection of the genus *Bacillus* (SEQ ID NO.1 and 3) and a capture oligonucleotide for detection of *Bacillus benzoevorans* (SEQ ID NO*.*30).

Table 11 shows the results for *Bacillus licheniformis.*

**Table 11 Test Bacteria: Bacillus licheniformis**

| Target Bacteria | SEQ ID NO./+ (signal present), - (signal absent) | | | | |
|---|---|---|---|---|---|
| Genus *Bacillus* | 1- | 2+ | 3+ | | |
| Genus *Brevibacillus* | 4- | | | | |
| Genus *Alicyclobacillus* | 5- | | | | |
| Genus *Aneurinibacillus* | 6- | | | | |
| Genus *Geobacillus* | 7- | | | | |
| Genus *Paenibacillus* | 8- | | | | |
| *Bacillus cereus* | 9- | 10- | 11- | | |
| *Bacillus coagulans* | 12- | 13- | 14- | | |
| *Bacillus sporothermodurans* | 15- | 43- | 44- | | |
| *Bacillus subtilis* | 16- | 17- | 18- | | |
| *Bacillus licheniformis* | 19+ | | | | |
| *Bacillus pumilus* | 20- | 55- | | | |
| *Bacillus lentus* | 21- | 22- | 23- | 24- | 25- |
| *Bacillus firmus* | 26- | | | | |
| *Bacillus circulans* | 27- | | | | |
| *Bacillus benzoevorans* | 29- | 30- | | | |
| *Bacillus megaterium* | 31- | | | | |
| *Bacillus sphaericus* | 32- | 33- | | | |
| *Alicyclobacillus acidoterrostris* | 34- | 35- | 36- | | |
| *Alicyclobacillus acidocaldarius* | 28- | 37- | | | |
| *Paenibacillus validus* | 38- | 39- | 40- | | |
| *Bacillus smithii* | 45- | 46- | | | |
| *Alicyclobacillus acidiphilus* | 47- | 48- | 49- | | |
| *Alicyclobacillus hesperidum* | 50- | 51- | | | |
| *Bacillus fumarioli* | 52- | 53- | | | |
| *Bacillus acidogenesis* | 54- | | | | |
| *Bacillus sonorensis* | 56- | 57- | | | |

According to Table 11, color generation was confirmed for *Bacillus licheniformis* at a total of three locations of capture oligonucleotides for detection of the genus *Bacillus* (SEQ ID NO. 2 and 3) and a capture oligonucleotide for detection of *Bacillus coagulans* (SEQ ID NO. 19).

Table 12 shows the results for *Alicyclobacillus acidoterrestris.*

**Table 12 Test Bacteria: Alicyclobacillus acidoterrestris**

| Target Bacteria | SEQ ID NO./+ (signal present), - (signal absent) | | | | |
|---|---|---|---|---|---|
| Genus *Bacillus* | 1- | 2- | 3- | | |
| Genus *Brevibacillus* | 4- | | | | |
| Genus *Alicuclobacillus* | 5+ | | | | |
| Genus *Aneurinibacillus* | 6- | | | | |
| Genus *Geobacillus* | 7- | | | | |
| Genus *Paenibacillus* | 8- | | | | |
| *Bacillus cereus* | 9- | 10- | 11- | | |
| *Bacillus coagulans* | 12- | 13- | 14- | | |
| *Bacillus sporothermodurans* | 15- | 43- | 44- | | |
| *Bacillus subtilis* | 16- | 17- | 18- | | |
| *Bacillus licheniformis* | 19- | | | | |
| *Bacillus pumilus* | 20- | 55- | | | |
| *Bacillus lentus* | 21- | 22- | 23- | 24- | 25- |
| *Bacillus firmus* | 26- | | | | |
| *Bacillus circulans* | 27- | | | | |
| *Bacillus benzoevorans* | 29- | 30- | | | |
| *Bacillus megaterium* | 31- | | | | |
| *Bacillus sphaericus* | 32- | 33- | | | |
| *Alicyclobacillus acidoterrestris* | 34- | 35+ | 36+ | | |
| *Alicyclobacillus acidocaldarius* | 28- | 37- | | | |
| *Paenibacillus validus* | 38- | 39- | 40- | | |
| *Bacillus smithii* | 45- | 46- | | | |
| *Alicyclobacillus acidiphilus* | 47- | 48- | 49- | | |
| *Alicyclobacillus hesperidum* | 50- | 51- | | | |
| *Bacillus fumaioli* | 52- | 53- | | | |
| *Bacillus acidogenesis* | 54- | | | | |
| *Bacillus sonorensis* | 56- | 57- | | | |

According to Table 12, color generation was confirmed for *Alicyclobacillus acidoterrestris* at a total of four locations of a capture oligonucleotide for detection of the genus *Alicyclobacillu* (SEQ ID NO. 5) and capture oligonucleotides for detection of *Alicyclobacillus acidoterrestris* (SEQ ID NO. 34, 35 and 36).

Table 13 shows the results for *Alicyclobacillus acidocaldarius*.

**Table 13 Test Bacteria: Alicyclobacillus acidocaldarius**

| Target Bacteria | SEQ ID NO./+ (signal present), - (signal absent) | | | | |
|---|---|---|---|---|---|
| Genus *Bacillus* | 1- | 2- | 3- | | |
| Genus *Brevibacillus* | 4- | | | | |
| Genus *Alicyclobacillus* | 5+ | | | | |
| Genus *Aneurinibacillus* | 6- | | | | |
| Genus *Geobacillus* | 7- | | | | |
| Genus *Paenibacillus* | 8- | | | | |
| *Bacillus cereus* | 9- | 10- | 11- | | |
| *Bacillus coagulans* | 12- | 13- | 14- | | |
| *Bacillus sporothermodurans* | 15- | 43- | 44- | | |
| *Bacillus subtilis* | 16- | 17- | 18- | | |
| *Bacillus licheniformis* | 19- | | | | |
| *Bacillus pumilus* | 20- | 55- | | | |
| *Bacillus lentus* | 21- | 22- | 23- | 24- | 25- |
| *Bacillus firmus* | 26- | | | | |
| *Bacillus circulans* | 27- | | | | |
| *Bacillus benzoevorans* | 29- | 30- | | | |
| *Bacillus megaterium* | 31- | | | | |
| *Bacillus sphaericus* | 32- | 33- | | | |
| *Alicyclobacillus acidoterrestris* | 34- | 35- | 36- | | |
| *Alicyclobacillus acidocaldarius* | 28+ | 37+ | | | |
| *Paenibacillus validus* | 38- | 39- | 40- | | |
| *Bacillus smithii* | 45- | 46- | | | |
| *Alicyclobacillus acidiphilus* | 47- | 48- | 49- | | |
| *Alicyclobacillus hesperidium* | 50- | 51- | | | |
| *Bacillus fumarioli* | 52- | 53- | | | |
| *Bacillus acidogenesis* | 54- | | | | |
| *Bacillus sonorensis* | 56- | 57- | | | |

According to Table 13, color generation was confirmed for *Alicyclobacillus acidocaldarius* at a total of three locations of a capture oligonucleotide for detection of the genus *Alicyclobacillus* (SEQ ID NO.5) and capture oligonucleotides for detection of *Alicyclobacillus acidocaldarius* (SEQ ID NO.28 and 37).

Table 14 shows the results for *Aneurinibacillus aneurinolyticus.*

**Table 14 Test Bacteria: Aneurinibacillus aneurinolyticus**

| Target Bacteria | SEQ ID NO./+ (signal present), - (signal absent) | | | | |
|---|---|---|---|---|---|
| Genus *Bacillus* | 1- | 2- | 3+ | | |
| Genus *Brevibacillus* | 4- | | | | |
| Genus *Alicyclobacillus* | 5- | | | | |
| Genus *Aneurinibacillus* | 6+ | | | | |
| Genus *Geobacillus* | 7- | | | | |
| Genus *Paenibacillus* | 8- | | | | |
| *Bacillus cereus* | 9- | 10- | 11- | | |
| *Bacillus coagulans* | 12- | 13- | 14- | | |
| *Bacillus sporothermodurans* | 15- | 43- | 44- | | |
| *Bacillus sublitis* | 16- | 17- | 18- | | |
| *Bacillus licheniformis* | 19- | | | | |
| *Bacillus pumilus* | 20- | 55- | | | |
| *Bacillus lentus* | 21- | 22- | 23- | 24- | 25- |
| *Bacillus firmus* | 26- | | | | |
| *Bacillus circulans* | 27- | | | | |
| *Bacillus benzoevorans* | 29- | 30- | | | |
| *Bacillus megaterium* | 31- | | | | |
| *Bacillus sphaericus* | 32- | 33- | | | |
| *Alicyclobacillus acidoterrestris* | 34- | 35- | 36- | | |
| *Alicyclobacillus acidocaldarius* | 28- | 37- | | | |
| *Paenibacillus validus* | 38- | 39- | 40- | | |
| *Bacillus smithii* | 45- | 46- | | | |
| *Alicyclobacillus acidiphilus* | 47- | 48- | 49- | | |
| *Alicyclobacillus hesperidum* | 50- | 51- | | | |
| *Bacillus fumarioli* | 52- | 53- | | | |
| *Bacillus acidogenesis* | 54- | | | | |
| *Bacillus sonorensis* | 56- | 57- | | | |

According to Table 14, color generation was confirmed for *Aneurinibacillus aneurinolyticus* at a total of two locations of a capture oligonucleotide for detection of the genus *Bacillus* (SEQ ID NO. 3) and a capture oligonucleotide for detection of *Aneurinibacillus* species (SEQ ID NO. 6).

Table 15 shows the results for *Geobacillus stearothermophilus.*

**Table 15 Test Bacteria: Geobacillus stearotermophilus**

| Target Bacteria | SEQ ID NO./+ (signal present), - (signal absent) | | | | |
|---|---|---|---|---|---|
| Genus *Bacillus* | 1+ | 2- | 3- | | |
| Genus *Brevibacillus* | 4- | | | | |
| Genus *Alicyclobacillus* | 5- | | | | |
| Genus *Aneurinibacillus* | 6- | | | | |
| Genus *Geobacillus* | 7+ | | | | |
| Genus *Paenibacillus* | 8- | | | | |
| *Bacillus cereus* | 9- | 10- | 11- | | |
| *Bacillus coagulans* | 12- | 13- | 14- | | |
| *Bacillus sporothermodurans* | 15- | 43- | 44- | | |
| *Bacillus subtilis* | 16- | 17- | 18- | | |
| *Bacillus licheniformis* | 19- | | | | |
| *Bacillus pumilus* | 20- | 55- | | | |
| *Bacillus lentus* | 21- | 22- | 23- | 24- | 25- |
| *Bacillus firmus* | 26- | | | | |
| *Bacillus circulans* | 27- | | | | |
| *Bacillus benzoevoans* | 29- | 30- | | | |
| *Bacillus megaterium* | 31- | | | | |
| *Bacillus sphaericus* | 32- | 33- | | | |
| *Alicyclobacillus acidoterrestris* | 34- | 35- | 36- | | |
| *Alicyclobacillus acidocaldarius* | 28- | 37- | | | |
| *Paenibacillus validus* | 38- | 39- | 40- | | |
| *Bacillus smithii* | 45- | 46- | | | |
| *Alicyclobacillus acidiphilus* | 47- | 48- | 49- | | |
| *Alicyclobacillus hesperidum* | 50- | 51- | | | |
| *Bacillus fumarioli* | 52- | 53- | | | |
| *Bacillus acidogenesis* | 54- | | | | |
| *Bacillus sonorensis* | 56- | 57- | | | |

According to Table 15, color generation was confirmed for *Geobacillus stearothermophilus* at a total of two locations of a capture oligonucleotide for detection of the genus *Bacillus* (SEQ ID NO.1) and a capture oligonucleotide for detection of *Geobacillus* species (SEQ ID NO. 7).

Table 16 shows the results *for Paenibacillus validus.*

**Table 16 Test Bacteria: Paenibacillus validus**

| Target Bacteria | SEQ ID NO./+ (signal present), - (signal absent) | | | | |
|---|---|---|---|---|---|
| Genus *Bacillus* | 1- | 2- | 3+ | | |
| Genus *Brevibacillus* | 4- | | | | |
| Genus *Alicyclobacillus* | 5- | | | | |
| Genus *Aneurinibacillus* | 6- | | | | |
| Genus *Geobacillus* | 7- | | | | |
| Genus *Paenibacillus* | 8+ | | | | |
| *Bacillus cereus* | 9- | 10- | 11- | | |
| *Bacillus coagulans* | 12- | 13- | 14- | | |
| *Bacillus sporothermodurans* | 15- | 43- | 44- | | |
| *Bacillus subtilis* | 16- | 17- | 18- | | |
| *Bacillus licheniformis* | 19- | | | | |
| *Bacillus pumilus* | 20- | 55- | | | |
| *Bacillus lentus* | 21- | 22- | 23- | 24- | 25- |
| *Bacillus firmus* | 26- | | | | |
| *Bacillus circulans* | 27- | | | | |
| *Bacillus benzoevorans* | 29- | 30- | | | |
| *Bacillus megaterium* | 31- | | | | |
| *Bacillus sphaericus* | 32- | 33- | | | |
| *Alicyclobacillus acidoterrestris* | 34- | 35- | 36- | | |
| *Alicyclobacillus acidocaldarius* | 28- | 37- | | | |
| *Paenibacillus validus* | 38+ | 39+ | 40+ | | |
| *Bacillus smithii* | 45- | 46- | | | |
| *Alicyclobacillus acidiphilus* | 47- | 48- | 49- | | |
| *Alicyclobacillus hesperidum* | 50- | 51- | | | |
| *Bacillus fumarioli* | 52- | 53- | | | |
| *Bacillus acidogenesis* | 54- | | | | |
| *Bacillus sonorensis* | 56- | 57- | | | |

According to Table 16, color generation was confirmed for *Paenibacillus validus* at a total of five locations of a capture oligonucleotide for detection of the genus *Bacillus* (SEQ ID NO. 3), a capture oligonucleotide for detection of *Paenibacillus* species (SEQ ID NO.8), and capture oligonucleotides for detection of *Paenibacillus validus* (SEQ ID NO. 38, 39 and 40).

Table 17 shows the results for *Bacillus cereus.*

**Table 17 Test Bacteria: Bacillus cereus**

| Target Bacteria | SEQ ID NO/+ (signal present), - (signal absent) | | | | |
|---|---|---|---|---|---|
| Genus *Bacillus* | 1- | 2- | 3- | | |
| Genus *Brevibacillus* | 4- | | | | |
| Genus *Alicyclobacillus* | 5- | | | | |
| Genus *Aneurinibacillus* | 6- | | | | |
| Genus *Geobacillus* | 7- | | | | |
| Genus *Paenibacillus* | 8- | | | | |
| *Bacillus cereus* | 9+ | 10+ | 11+ | | |
| *Bacillus coagulans* | 12- | 13- | 14- | | |
| *Bacillus sporothermodurans* | 15- | 43- | 44- | | |
| *Bacillus subtilis* | 16- | 17- | 18- | | |
| *Bacillus licheniformis* | 19- | | | | |
| *Bacillus pumilus* | 20- | 55- | | | |
| *Bacillus lentus* | 21- | 22- | 23- | 24- | 25- |
| *Bacillus firmus* | 26- | | | | |
| *Bacillus circulans* | 27- | | | | |
| *Bacilus benzoevorans* | 29- | 30- | | | |
| *Bacillus megaterium* | 31- | | | | |
| *Bacillus sphaericus* | 32- | 33- | | | |
| *Alicyclobacillus acidoterrestris* | 34- | 35- | 36- | | |
| *Alicyclobacillus acidocaldarius* | 28- | 37- | | | |
| *Paenibacillus validus* | 38- | 39- | 40- | | |
| *Bacillus smithii* | 45- | 46- | | | |
| *Alicyclobacillus acidiphilus* | 47- | 48- | 49- | | |
| *Alicyclobacillus hesperidum* | 50- | 51- | | | |
| *Bacillus fumarioli* | 52- | 53- | | | |
| *Bacillus acidogenesis* | 54- | | | | |
| *Bacillus sonorensis* | 56- | 57- | | | |

According to Table 17, color generation was confirmed for *Bacillus cereus* at a total of three locations of capture oligonucleotides for detection of *Bacillus cereus* (SEQ ID NO.9, 10 and 11).

Table 18 shows the results for *Paenibacillus illinoisensis.*

**Table 18 Test Bacteria: Paenibacillus illinoisensis**

| Target Bacteria | SEQ ID NO/+ (signal present), - (signal absent) | | | | |
|---|---|---|---|---|---|
| Genus *Bacillus* | 1- | 2- | 3- | | |
| Genus *Brevibacillus* | 4- | | | | |
| Genus *Alicyclobacillus* | 5- | | | | |
| Genus *Aneurinibacillus* | 6- | | | | |
| Genus *Geobacillus* | 7- | | | | |
| Genus *Paenibacillus* | 8+ | | | | |
| *Bacillus cereus* | 9- | 10- | 11- | | |
| *Bacillus coagulans* | 12- | 13- | 14- | | |
| *Bacillus sporothermodurans* | 15- | 43- | 44- | | |
| *Bacillus subtilis* | 16- | 17- | 18- | | |
| *Bacillus licheniformis* | 19- | | | | |
| *Bacillus pumilus* | 20- | 55- | | | |
| *Bacillus lentus* | 21- | 22- | 23- | 24- | 25- |
| *Bacillus firmus* | 26- | | | | |
| *Bacillus circulans* | 27- | | | | |
| *Bacillus benzoevorans* | 29- | 30- | | | |
| *Bacillus megaterium* | 31- | | | | |
| *Bacillus sphaericus* | 32- | 33- | | | |
| *Alicyclobacillus acidoterrestris* | 34- | 35- | 36- | | |
| *Alicyclobacillus acidocaldarius* | 28- | 37- | | | |
| *Paenibacillus validus* | 38- | 39- | 40- | | |
| *Bacillus smithii* | 45- | 46- | | | |
| *Alicyclobacillus acidiphilus* | 47- | 48- | 49- | | |
| *Alicyclobacillus hesperidum* | 50- | 51- | | | |
| *Bacillus fumarioli* | 52- | 53- | | | |
| *Bacillus acidogenesis* | 54- | | | | |
| *Bacillus sonorensis* | 56- | 57- | | | |

According to Table 18, color generation was confinned for *Paenibacillus illinoisensis* at one location of a capture oligonucleotide for detection of the genus *Paenibacillus* (SEQ ID NO.8).

Table 19 shows the results for *Paenibacillus alvei.*

**Table 19 Test Bacteria: Paenibacillus alvei**

| Target Bacteria | SEQ ID NO./+ (signal present), - (signal absent) | | | | |
|---|---|---|---|---|---|
| Genus *Bacillus* | 1- | 2- | 3- | | |
| Genus *Brevibacillus* | 4- | | | | |
| Genus *Alicyclobacillus* | 5- | | | | |
| Genus *Aneurinibacillus* | 6- | | | | |
| Genus *Geobacillus* | 7- | | | | |
| Genus *Paenibacillus* | 8+ | | | | |
| *Bacillus cereus* | 9- | 10- | 11- | | |
| *Bacillus coagulans* | 12- | 13- | 14- | | |
| *Bacillus sporothermodurans* | 15- | 43- | 44- | | |
| *Bacillus subtilis* | 16- | 17- | 18- | | |
| *Bacillus licheniformis* | 19- | | | | |
| *Bacillus pumilus* | 20- | 55- | | | |
| *Bacillus lentus* | 21- | 22- | 23- | 24- | 25- |
| *Bacillus firmus* | 26- | | | | |
| *Bacillus circulans* | 27- | | | | |
| *Bacillus benzoevorans* | 29- | 30- | | | |
| *Bacillus megaterium* | 31- | | | | |
| *Bacillus sphaericus* | 32- | 33- | | | |
| *Alicyclobacillus acidoterrestris* | 34- | 35- | 36- | | |
| *Alicyclobacillus acidocaldarius* | 28- | 37- | | | |
| *Paenibacillus validus* | 38- | 39- | 40- | | |
| *Bacillus smithii* | 45- | 46- | | | |
| *Alicyclobacillus acidiphilus* | 47- | 48- | 49- | | |
| *Alicyclobacillus hesperidum* | 50- | 51- | | | |
| *Bacillus fumarioli* | 52- | 53- | | | |
| *Bacillus acidogenesis* | 54- | | | | |
| *Bacillus sonorensis* | 56- | 57- | | | |

According to Table 19, color generation was confirmed for *Paenibacillus alvei* at one location of a capture oligonucleotide for detection of the genus *Paenibacillus* (SEQ ID NO. 8).

Table 20 shows the results for *Paenibacillus polymyxa.*

**Table 20 Test Bacteria: Paenibacillus polymyxa**

| Target Bacteria | SEQ ID NO./+ (signal present), - (signal absent) | | | | |
|---|---|---|---|---|---|
| Genus *Bacillus* | 1- | 2- | 3- | | |
| Genus *Brevibacillus* | 4- | | | | |
| Genus *Alicyclobacillus* | 5- | | | | |
| Genus *Aneurinibacillus* | 6- | | | | |
| Genus *Geobacillus* | 7- | | | | |
| Genus *Paenibacillus* | 8+ | | | | |
| *Bacillus cereus* | 9- | 10- | 11- | | |
| *Bacillus coagulans* | 12- | 13- | 14- | | |
| *Bacillus spororhermodurans* | 15- | 43- | 44- | | |
| *Bacillus subtilis* | 16- | 17- | 18- | | |
| *Bacillus licheniformis* | 19- | | | | |
| *Bacillus pumilus* | 20- | 55- | | | |
| *Bacillus lentus* | 21- | 22- | 23- | 24- | 25- |
| *Bacillus firmus* | 26- | | | | |
| *Bacillus circulans* | 27- | | | | |
| *Bacillus benzoevorans* | 29- | 30- | | | |
| *Bacillus megaterium* | 31- | | | | |
| *Bacillus sphaericus* | 32- | 33- | | | |
| *Alicyclobacillus acidoterrestris* | 34- | 35- | 36- | | |
| *Alicyclobacillus acidocaldarius* | 28- | 37- | | | |
| *Paenibacillus validus* | 38- | 39- | 40- | | |
| *Bacillus smithii* | 45- | 46- | | | |
| *Alicyclobacillus acidiphilus* | 47- | 48- | 49- | | |
| *Alicyclobacillus hesperidum* | 50- | 51- | | | |
| *Bacillus fumarioli* | 52- | 53- | | | |
| *Bacillus acidogenesis* | 54- | | | | |
| *Bacillus sonorensis* | 56- | 57- | | | |

According to Table 20, color generation was confirmed for *Paenibacillus polymyxa* at one location of a capture oligonucleotide for detection of the genus *Paenibacillus* (SEQ ID NO.8).

Table 21 shows the results for *Brevibacillus brevis.*

**Table 21 Test Bacteria: Brevibacillus brevis**

| Target Bacteria | SEQ ID NO./+ (signal present), - (signal absent) | | | | |
|---|---|---|---|---|---|
| Genus *Bacillus* | 1- | 2- | 3- | | |
| Genus *Brevibacillus* | 4+ | | | | |
| Genus *Alicyclobacillus* | 5- | | | | |
| Genus *Aneurinibacillus* | 6- | | | | |
| Genus *Geobacillus* | 7- | | | | |
| Genus *Paenibacillus* | 8- | | | | |
| *Bacillus cereus* | 9- | 10- | 11- | | |
| *Bacillus coagulans* | 12- | 13- | 14- | | |
| *Bacillus sporothermodurans* | 15- | 43- | 44- | | |
| *Bacillus subtilis* | 16- | 17- | 18- | | |
| *Bacillus licheniformis* | 19- | | | | |
| *Bacillus pumilus* | 20- | 55- | | | |
| *Bacillus lentus* | 21- | 22- | 23- | 24- | 25- |
| *Bacillus firmus* | 26- | | | | |
| *Bacillus circulans* | 27- | | | | |
| *Bacillus benzoevorans* | 29- | 30- | | | |
| *Bacillus megaterium* | 31- | | | | |
| *Bacillus sphaericus* | 32- | 33- | | | |
| *Alicyclobacillus acidoterrestris* | 34- | 35- | 36- | | |
| *Alicyclobacillus acidocaldarius* | 28- | 37- | | | |
| *Paenibacillus validus* | 38- | 39- | 40- | | |
| *Bacillus smithii* | 45- | 46- | | | |
| *Alicyclobacillus acidiphilus* | 47- | 48- | 49- | | |
| *Alicyclobacillus hesperidum* | 50- | 51- | | | |
| *Bacillus fumarioli* | 52- | 53- | | | |
| *Bacillus acidogenesis* | 54- | | | | |
| *Bacillus sonorensis* | 56- | 57- | | | |

According to Table 21, color generation was confirmed for *Brevibacillus brevis* at one location of a capture oligonucleotide for detection of *Brevibacillus* species (SEQ ID NO.4).

Table 22 shows the results for *Bacillus smithii.*

**Table 22 Test Bacteria: Bacillus smithii**

| Target Bacteria | SEQ ID NO./*+* (signal present), - (signal absent) | | | | |
|---|---|---|---|---|---|
| Genus *Bacillus* | 1+ | 2- | 3+ | | |
| Genus *Brevibacillus* | 4- | | | | |
| Genus *Alicyclobacillus* | 5- | | | | |
| Genus *Aneurinibacillus* | 6- | | | | |
| Genus *Geobacillus* | 7- | | | | |
| Genus *Paenibacillus* | 8- | | | | |
| *Bacillus cereus* | 9- | 10- | 11- | | |
| *Bacillus coagulans* | 12- | 13- | 14- | | |
| *Bacillus sporothermodurans* | 15- | 43- | 44- | | |
| *Bacillus subtilis* | 16- | 17- | 18- | | |
| *Bacillus licheniformis* | 19- | | | | |
| *Bacillus pumilus* | 20- | 55- | | | |
| *Bacillus lentus* | 21- | 22- | 23- | 24- | 25- |
| *Bacillus firmus* | 26- | | | | |
| *Bacillus circulans* | 27- | | | | |
| *Bacillus benzoevorans* | 29- | 30- | | | |
| *Bacillus megaterium* | 31- | | | | |
| *Bacillus sphaericus* | 32- | 33- | | | |
| *Alicyclobacillus acidoterrestris* | 34- | 35- | 36- | | |
| *Alicyclobacillus acidocaldarius* | 28- | 37- | | | |
| *Paenibacillus validus* | 38- | 39- | 40- | | |
| *Bacillus smithii* | 45+ | 46+ | | | |
| *Alicyclobacillus acidiphilus* | 47- | 48- | 49- | | |
| *Alicyclobacillus hesperidum* | 50- | 51- | | | |
| *Bacillus fumarioli* | 52- | 53- | | | |
| *Bacillus acidogenesis* | 54- | | | | |
| *Bacillus sonorensis* | 56- | 57- | | | |

According to Table 22, color generation was confirmed for *Bacillus* smithii at a total of four locations of capture oligonucleotides for detection of the genus *Bacillus* (SEQ ID NO. 1 and 3) and capture oligonucleotides for detection of *Bacillus smithii* (SEQ ID NO. 45 and 46).

Table 23 shows the results for *Alicyclobacillus acidiphilus.*

**Table 23 Test Bacteria: Alicyclobacillus acidiphilus**

| Target Bacteria | SEQ ID NO./+ (signal present), - (signal absent) | | | | |
|---|---|---|---|---|---|
| Genus *Bacillus* | 1- | 2- | 3- | | |
| Genus *Brevibacillus* | 4- | | | | |
| Genus *Alicyclobacillus* | 5+ | | | | |
| Genus *Aneurinibacillus* | 6- | | | | |
| Genus *Geobacillus* | 7- | | | | |
| Genus *Paenibacillus* | 8- | | | | |
| *Bacillus cereus* | 9- | 10- | 11- | | |
| *Bacillus coagulans* | 12- | 13- | 14- | | |
| *Bacillus sporothermodurans* | 15- | 43- | 44- | | |
| *Bacillus subtilis* | 16- | 17- | 18- | | |
| *Bacillus licheniformis* | 19- | | | | |
| *Bacillus pumilus* | 20- | 55- | | | |
| *Bacillus lentus* | 21- | 22- | 23- | 24- | 25- |
| *Bacillus firmus* | 26- | | | | |
| *Bacillus circulans* | 27- | | | | |
| *Bacillus benzoevorans* | 29- | 30- | | | |
| *Bacillus megaterium* | 31- | | | | |
| *Bacillus sphaericus* | 32- | 33- | | | |
| *Alicyclobacillus acidoterrestris* | 34- | 35- | 36- | | |
| *Alicyclobacillus acidocaldarius* | 28- | 37- | | | |
| *Paenibacillus validus* | 38- | 39- | 40- | | |
| *Bacillus smithii* | 45- | 46- | | | |
| *Alicyclobacillus acidiphilus* | 47+ | 48+ | 49+ | | |
| *Alicyclobacillus hesperidum* | 50- | 51- | | | |
| *Bacillus fumarioli* | 52- | 53- | | | |
| *Bacillus acidogenesis* | 54- | | | | |
| *Bacillus sonorensis* | 56- | 57- | | | |

According to Table 23, color generation was confirmed for *Alicyclobacillus acidiphilus* at a total of four locations of a capture oligonucleotide for detection of the genus *Alicyclobacillus* (SEQ ID NO.5) and capture oligonucleotides for detection of *Alicyclobacillus acidiphilus* (SEQ ID NO. 47 to 49).

Table 24 shows the results for *Alicyclobacillus hesperidum.*

**Table 24 Test Bacteria: Alicyclobacillus hesperidum**

| Target Bacteria | SEQ ID NO./+ (signal present), - (signal absent) | | | | |
|---|---|---|---|---|---|
| Genus *Bacillus* | 1- | 2- | 3- | | |
| Genus *Brevibacillus* | 4- | | | | |
| Genus *Alicyclobacillus* | 5+ | | | | |
| Genus *Aneurinibacillus* | 6- | | | | |
| Genus *Geobacillus* | 7- | | | | |
| Genus *Paenibacillus* | 8- | | | | |
| *Bacillus cereus* | 9- | 10- | 11- | | |
| *Bacillus coagulans* | 12- | 13- | 14- | | |
| *Bacillus sporothermodurans* | 15- | 43- | 44- | | |
| *Bacillus subtilis* | 16- | 17- | 18- | | |
| *Bacillus licheniformis* | 19- | | | | |
| *Bacillus pumilus* | 20- | 55- | | | |
| *Bacillus lentus* | 21- | 22- | 23- | 24- | 25- |
| *Bacillus firmus* | 26- | | | | |
| *Bacillus circulans* | 27- | | | | |
| *Bacillus benzoevorans* | 29- | 30- | | | |
| *Bacillus megaterium* | 31- | | | | |
| *Bacillus sphaericus* | 32- | 33- | | | |
| *Alicyclobacillus acidoterrestris* | 34- | 35- | 36- | | |
| *Alicyclobacillus acidocaldarius* | 28- | 37- | | | |
| *Paenibacillus validus* | 38- | 39- | 40- | | |
| *Bacillus smithii* | 45- | 46- | | | |
| *Alicyclobacillus acidiphilus* | 47- | 48- | 49- | | |
| *Alicyclobacillus hesperidum* | 50+ | 51+ | | | |
| *Bacillus fumarioli* | 52- | 53- | | | |
| *Bacillus acidogenesis* | 54- | | | | |
| *Bacillus sonorensis* | 56- | 57- | | | |

According to Table 24, color generation was confirmed for *Alicyclobacillus hesperidum* at a total of three locations of a capture oligonucleotide for detection of the genus *Alicyclobacillus* (SEQ ID NO. 5) and capture oligonucleotides for detection of *Alicyclobacillus hesperidum* (SEQ ID NO. 50 and 51).

Table 25 shows the results for *Bacillus fumarioli.*

**Table 25 Test Bacteria: Bacillus fumarioli**

| Target Bacteria | SEQ ID NO./+ (signal present), - (signal absent) | | | | |
|---|---|---|---|---|---|
| Genus *Bacillus* | 1+ | 2- | 3+ | | |
| Genus *Brevibacillus* | 4- | | | | |
| Genus *Alicyclobacillus* | 5- | | | | |
| Genus *Aneurinibacillus* | 6- | | | | |
| Genus *Geobacillus* | 7- | | | | |
| Genus *Paenibacillus* | 8- | | | | |
| *Bacillus cereus* | 9- | 10- | 11- | | |
| *Bacillus coagulans* | 12- | 13- | 14- | | |
| *Bacillus sporothermodurans* | 15- | 43- | 44- | | |
| *Bacillus subtilis* | 16- | 17- | 18- | | |
| *Bacillus licheniformis* | 19- | | | | |
| *Bacillus pumilus* | 20- | 55- | | | |
| *Bacillus lentus* | 21- | 22- | 23- | 24- | 25- |
| *Bacillus firmus* | 26- | | | | |
| *Bacillus circulans* | 27- | | | | |
| *Bacillus benzoevorans* | 29- | 30- | | | |
| *Bacillus megaterium* | 31- | | | | |
| *Bacillus sphaericus* | 32- | 33- | | | |
| *Alicyclobacillus acidoterrestris* | 34- | 35- | 36- | | |
| *Alicyclobacillus acidocaldarius* | 28- | 37- | | | |
| *Paenibacillus validus* | 38- | 39- | 40- | | |
| *Bacillus smithii* | 45- | 46- | | | |
| *Alicyclobacillus acidiphilus* | 47- | 48- | 49- | | |
| *Alicyclobacillus hesperidum* | 50- | 51- | | | |
| *Bacillus fumarioli* | 52+ | 53+ | | | |
| *Bacillus acidogenesis* | 54- | | | | |
| *Bacillus sonosensis* | 56- | 57- | | | |

According to Table 25, color generation was confirmed for *Bacillus fumarioli* at a total of four locations of capture oligonucleotides for detection of the genus *Bacillus* (SEQ ID NO. 1 and 3) and capture oligonucleotides for detection of *Bacillus fumarioli* (SEQ ID NO. 52 and 53).

Table 26 shows the results for *Bacillus acidogenesis.*

**Table 26 Test Bacteria: Bacillus acidogenesis**

| Target Bacteria | SEQ ID NO./+ (signal present), - (signal absent) | | | | |
|---|---|---|---|---|---|
| Genus *Bacillus* | 1+ | 2+ | 3+ | | |
| Genus *Brevibocellus* | 4- | | | | |
| Genus *Alicyclobacillus* | 5- | | | | |
| Genus *Aneurinibacillus* | 6- | | | | |
| Genus *Geobacillus* | 7- | | | | |
| Genus *Paenibacillus* | 8- | | | | |
| *Bacillus cereus* | 9- | 10- | 11- | | |
| *Bacillus coagulans* | 12- | 13- | 14- | | |
| *Bacillus sporothermodurans* | 15- | 43- | 44- | | |
| *Bacillus subtilis* | 16- | 17- | 18- | | |
| *Bacillus licheniformis* | 19- | | | | |
| *Bacillus pumillus* | 20- | 55- | | | |
| *Bacillus lentus* | 21- | 22- | 23- | 24- | 25- |
| *Bacillus firmus* | 26- | | | | |
| *Bacillus circulans* | 27- | | | | |
| *Bacillus benzoevorans* | 29- | 30- | | | |
| *Bacillus megaterium* | 31- | | | | |
| *Bacillus sphaericus* | 32- | 33- | | | |
| *Alicyclobacillus acidoterrestris* | 34- | 35- | 36- | | |
| *Alicyclobacillus acidocaldarius* | 28- | 37- | | | |
| *Paenibacillus validus* | 38- | 39- | 40- | | |
| *Bacillus smithii* | 45- | 46- | | | |
| *Alicyclobacillus acidiphilus* | 47- | 48- | 49- | | |
| *Alicyclobacillus hesperidum* | 50- | 51- | | | |
| *Bacillus fumarioli* | 52- | 53- | | | |
| *Bacillus acidogenesis* | 54+ | | | | |
| *Bacillus sonorensis* | 56- | 57- | | | |

According to Table 26, color generation was confirmed for *Bacillus acidogenesis* at a total of four locations of capture oligonucleotides for detection of the genus *Bacillus* (SEQ ID NO. 1 to 3) and a capture oligonucleotide for detection of *Bacillus acidogenesis* (SEQ ID NO. 54).

Table 27 shows the results for *Bacillus sonorensis.*

**Table 27 Test Bacteria: Bacillus sonorensis**

| Target Bacteria | SEQ ID NO./+ (signal present), - (signal absent) | | | | |
|---|---|---|---|---|---|
| Genus *Bacillus* | 1+ | 2+ | 3+ | | |
| Genus *Brevibacillus* | 4- | | | | |
| Genus *Alicyclobacillus* | 5- | | | | |
| Genus *Aneurinibacillus* | 6- | | | | |
| Genus *Geobacillus* | 7- | | | | |
| Genus *Paenibacillus* | 8- | | | | |
| *Bacillus cereus* | 9- | 10- | 11- | | |
| *Bacillus coagulans* | 12- | 13- | 14- | | |
| *Bacillus sporothermodurans* | 15- | 43- | 44- | | |
| *Bacillus subtilis* | 16- | 17- | 18- | | |
| *Bacillus licheniformis* | 19- | | | | |
| *Bacillus pumilus* | 20- | 55- | | | |
| *Bacillus lentus* | 21- | 22- | 23- | 24- | 25- |
| *Bacillus firmus* | 26- | | | | |
| *Bacillus circulans* | 27- | | | | |
| *Bacillus benzoevorans* | 29- | 30- | | | |
| *Bacillus megaterium* | 31- | | | | |
| *Bacillus sphaericus* | 32- | 33- | | | |
| *Alicyclobacillus acidoterrestris* | 34- | 35- | 36- | | |
| *Alicyclobacillus acidocaldarius* | 28- | 37- | | | |
| *Paenibacillus validus* | 38- | 39- | 40- | | |
| *Bacillus smithii* | 45- | 46- | | | |
| *Alicyclobacillus acidiphilus* | 47- | 48- | 49- | | |
| *Alicyclobacillus hesperidum* | 50- | 51- | | | |
| *Bacillus fumarioli* | 52- | 53- | | | |
| *Bacillus acidogenesis* | 54- | | | | |
| *Bacillus sonorensis* | 56+ | 57+ | | | |

According to Table 27, color generation was confirmed for *Bacillus sonorensis* at a total of five locations of capture oligonucleotides for detection of the genus *Bacillus* (SEQ ID NO. 1 to 3) and capture oligonucleotides for detection of *Bacillus sonorensis* (SEQ ID NO. 56 and 57).

In addition, color generation was not obtained for any of the capture oligonucleotides from control bacteria consisting of *Escherichia coli, Staphylococcus aureus, Staphylococcus capitis, Streptococcus salivarius, Pseudomonas putida* and *Lactobacillus delbrueckii.*

On the basis of these results, a bacteria detection device (substrate) of the present embodiment was confirmed to be capable of specifically detecting the genus *Bacillus,* the genus *Brevibacillus,* the genus *Alicyclobacillus,* the genus *Aneurinibacillus,* the genus *Geobacillus* and the genus *Paenibacillus,* and species-specifically differentiating bacteria such as *Bacillus cereus, Bacillus coagulans, Bacillus subtilis, Bacillus licheniformis, Paenibacillus validus, Bacillus pumilus, Bacillus megaterium, Bacillus sphaericus, Alicyclobacillus acidoterrestris, Alicyclobacillus acidocaldarius, Bacillus lentus, Bacillus firmus, Bacillus circulans, Bacillus benzoevorans, Bacillus licheniformis, Bacillus smithii, Bacillus fumarioli, Bacillus acidogenesis, Bacillus sonorensis, Alicyclobacillus acidiphilus and Alicyclobacillus hesperidum.*

### INDUSTRIAL APPLICABILITY

The present invention provides a bacteria detection device, bacteria detection method and bacteria detection kit enabling rapid and accurate determination of bacteria present in a test sample with a simple procedure. Thus, the present invention can be used for sanitary control, process control and quality control in the food production industry, food industry, pharmaceutical industry and so on.

## Claims

1. A bacteria detection device for detecting and identifying bacteria belonging to the genus Bacillus and/or Bacillus species in a test sample, wherein an oligonucleotide consisting of a nucleotide sequence of SEQ ID NO:1, an oligonucleotide consisting of the nucleotide sequence of SEQ ID NO:2, and an oligonucleotide consisting of the nucleotide sequence of SEQ ID NO:3 among nucleotide sequences corresponding to a 16S ribosomal RNA gene of bacteria targeted for detection, are immobilized on a substrate; wherein said bacteria detection device is for detecting and identifying said bacteria in a test sample by hybridization between the oligonucleotide and a nucleic acid derived from the test sample.

2. The bacteria detection device according to claim1,
wherein the device further comprises:
(a) at least one of the oligonucleotides consisting of the nucleotide sequence of SEQ ID NO:9, 10 or 11, for detection of *Bacillus cereus;* and
(b) at least one of the oligonucleotides consisting of the nucleotide sequence of SEQ ID NO:15, 43 or 44, for detection of *Bacillus sporothermodurans.*

3. The bacteria detection device according to claim 1 or 2, wherein the device has a carbodiimide group or an isocyanate group on the substrate, and a covalent bond is formed as a result of a reaction between the carbodiimide group or the isocyanate group and the oligonucleotide or a linker added to a terminal of the oligonucleotide.

4. A bacteria detection method for detecting and identifying bacteria belonging to the genus Bacillus and/or Bacillus species in a test sample, comprising the steps of:
preparing a nucleic acid of the bacteria in the test sample;
preparing a labeled probe using the nucleic acid as a template;
hybridizing the labeled probe with an oligonucleotide immobilized on a substrate using the bacteria detection device according to any of claims 1 to 3; and
detecting a hybridization signal.

5. The bacteria detection method according to claim 4, wherein the test sample is a food.

6. The bacteria detection method according to claim 5 wherein the food is a beverage.

7. The bacteria detection method according to claim 6 wherein the food is a refreshing beverage.

8. A bacteria detection kit for carrying out the bacteria detection method according to any of claims 4 to 7, wherein the kit comprises the bacteria deletion device according to any of claims 1 to 3.

## Patentansprüche

1. Bakteriennachweis-Vorrichtung zum Nachweis und zur Identifizierung von Bakterien, die zur Gattung Bacillus und/oder der Spezies Bacillus gehören, in einer Testprobe, wobei ein Oligonucleotid bestehend aus einer Nucleotidsequenz der SEQ ID NO:1, ein Oligonucleotid bestehend aus der Nucleotidsequenz der SEQ ID NO:2 und ein Oligonucleotid bestehend aus der Nucleotidsequenz der SEQ ID NO:3 unter den Nucleotidsequenzen entsprechend einem 16S ribosomalen RNA-Gen von Bakterien als Ziel für den Nachweis auf einem Substrat immobilisiert sind; wobei die Bakteriennachweis-Vorrichtung zum Nachweis und zur Identifizierung der Bakterien in einer Testprobe durch Hybridisierung zwischen dem Oligonucleotid und einer Nucleinsäure, die aus der Testprobe stammt, dient.

2. Bakteriennachweis-Vorrichtung nach Anspruch 1, wobei die Vorrichtung außerdem umfasst:
(a) mindestens eines der Oligonucleotide bestehend aus der Nucleotidsequenz der SEQ ID NO:9, 10 oder 11, zum Nachweis von *Bacillus cereus;* und
(b) mindestens eines der Oligonucleotide bestehend aus der Nucleotidsequenz der SEQ ID NO:15, 43 oder 44, zum Nachweis von *Bacillus sporothermodurans.*

3. Bakteriennachweis-Vorrichtung nach Anspruch 1 oder 2, wobei die Vorrichtung eine Carbodiimidgruppe oder eine Isocyanatgruppe auf dem Substrat hat, und eine kovalente Bindung als Resultat einer Reaktion zwischen der Carbodiimidgruppe oder der Isocyanatgruppe und dem Oligonucleotid oder einem Linker, der an ein Ende des Oligonucleotids angefügt ist, gebildet wird.

4. Bakteriennachweisverfahren zum Nachweis und zur Identifizierung von Bakterien, die zur Gattung Bacillus und/oder der Spezies Bacillus gehören, in einer Testprobe, das die Schritte umfasst: Herstellen einer Nucleinsäure von den Bakterien in der Testprobe; Herstellen einer markierten Sonde unter Verwendung der Nucleinsäure als Matrize; Hybridisieren der markierten Sonde mit einem Oligonucleotid, das auf einem Substrat immobilisiert ist, unter Verwendung der Bakteriennachweis-Vorrichtung nach einem der Ansprüche 1 bis 3; und Nachweis eines Hybridisierungssignals.

5. Bakteriennachweisverfahren nach Anspruch 4, wobei die Testprobe ein Lebensmittel ist.

6. Bakteriennachweisverfahren nach Anspruch 5 wobei das Lebensmittel ein Getränk ist.

7. Bakteriennachweisverfahren nach Anspruch 6 wobei das Lebensmittel ein Erfrischungsgetränk ist.

8. Bakteriennachweis-Kit zur Durchführung des Bakteriennachweisverfahrens nach einem der Ansprüche 4 bis 7, wobei das Kit die Bakteriennachweis-Vorrichtung nach einem der Ansprüche 1 bis 3 umfasst.

## Revendications

1. Dispositif de détection de bactéries destiné à détecter et à identifier des bactéries appartenant au genre Bacillus et/ou à l'espèce Bacillus dans un échantillon de test, dans lequel un oligonucléotide consistant en une séquence de nucléotides de SEQ ID NO: 1, un oligonucléotide consistant en la séquence de nucléotides de SEQ ID NO: 2, et un oligonucléotide consistant en la séquence de nucléotides de SEQ ID NO: 3 parmi des séquences de nucléotides correspondant à un gène d'ARN ribosomique 16S des bactéries ciblées pour la détection, sont immobilisés sur un substrat ; ledit dispositif de détection de bactéries étant destiné à détecter et à identifier lesdites bactéries dans un échantillon de test par une hybridation entre l'oligonucléotide et un acide nucléique dérivé de l'échantillon de test.

2. Dispositif de détection de bactéries selon la revendication 1, le dispositif comprenant en outre :
(a)au moins un des oligonucléotides consistant en la séquence de nucléotides de SEQ ID NO: 9, 10 ou 11, pour la détection du *Bacillus cereus ;* et
(b)au moins un des oligonucléotides consistant en la séquence de nucléotides de SEQ ID NO: 15, 43 ou 44, pour la détection du *Bacillus sporothermodurans.*

3. Dispositif de détection de bactéries selon la revendication 1 ou 2, le dispositif possédant un groupe carbodiimide ou un groupe isocyanate sur le substrat, et une liaison covalente est formée en conséquence d'une réaction entre le groupe carbodiimide ou le groupe isocyanate et l'oligonucléotide ou une séquence de liaison ajoutée à une terminaison de l'oligonucléotide.

4. Procédé de détection de bactéries destiné à détecter et à identifier des bactéries appartenant au genre Bacillus et/ou à l'espèce Bacillus dans un échantillon de test, comprenant les étapes consistant à :
préparer un acide nucléique des bactéries dans l'échantillon de test ;
préparer une sonde marquée en utilisant l'acide nucléique en tant que matrice ;
hybrider la sonde marquée avec un oligonucléotide immobilisé sur un substrat en utilisant le dispositif de détection de bactéries selon l'une quelconque des revendications 1 à 3 ; et
détecter un signal d'hybridation.

5. Procédé de détection de bactéries selon la revendication 4, dans lequel l'échantillon de test est un aliment.

6. Procédé de détection de bactéries selon la revendication 5, dans lequel l'aliment est une boisson.

7. Procédé de détection de bactéries selon la revendication 6, dans lequel l'aliment est une boisson rafraîchissante.

8. Kit de détection de bactéries pour mettre en oeuvre le procédé de détection de bactéries selon l'une quelconque des revendications 4 à 7, le kit comprenant le dispositif de détection de bactéries selon l'une quelconque des revendications 1 à 3.
